(19) Europäisches Patentamt
European Patent Office
Office européen des brevets

(11) Publication number: 0 550 019 A2

# (12) EUROPEAN PATENT APPLICATION

(21) Application number: 92121933.3

(22) Date of filing: 23.12.92

(51) Int. Cl.[5]: C07D 401/04, C07D 215/56, C07D 471/04, A61K 31/47

(30) Priority: 31.12.91 KR 25886

(43) Date of publication of application: 07.07.93 Bulletin 93/27

(84) Designated Contracting States: AT BE CH DE DK ES FR GB GR IE IT LI LU NL PT SE

(71) Applicant: KOREA RESEARCH INSTITUTE OF CHEMICAL TECHNOLOGY
100, Jang-Dong, Yuseong-Ku
Daejeon, 305-606(KR)

(72) Inventor: Kim, Wan Joo
383-7 Dorhyong-dong, Yuseng-ku
Daejeon 305-606(KR)
Inventor: Park, Myung Hwan
431 Dorhyong-dong, Yuseong-ku
Daejeon 305-606(KR)
Inventor: Ha, Jae Du
100 Jang-dong, Yuseong-ku
Daejeon 305-606(KR)
Inventor: Baik, Kyong Up
39-35 Gajang-dong, Seo-ku
Daejeon 302-181(KR)
Inventor: Lee, Tae Suk
Samchang Apt. 1-203, Gajang-dong, Seo-ku
Daejeon 302-181(KR)
Inventor: Park, Tae Ho
431-6 Dorhong-dong, Yuseong-ku
Daejeon 305-606(KR)
Inventor: Nam, Keun Soo
57-50 Gajang-dong, Seo-ku
Daejeon 302-181(KR)
Inventor: Kim, Bong Jim
405-16 Kung-dong, Yuseong-ku
Daejeon 305-606(KR)

(74) Representative: Becker, Heinrich Karl Engelbert, Dr. et al
HOECHST AKTIENGESELLSCHAFT Central Patent Department P.O. Box 80 03 20
W-6230 Frankfurt am Main 80 (DE)

## (54) Novel quinolone carboxylic acid derivatives and processes for preparing same.

(57) The present invention relates to certain novel quinolone compounds of the present invention represented by the following formula(I) and novel processes for preparing same.

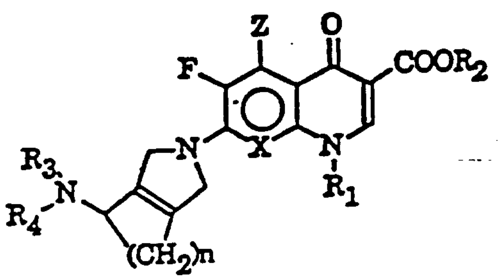

(I)

wherein:

X is a nitrogen atom or a C-Y group wherein Y is a hydrogen, fluorine, chlorine or bromine atom or a methoxy or methyl group;

EP 0 550 019 A2

$R_1$ is a $C_{1-6}$ alkyl group optionally substituted with a halogen atom or a hydroxy radical, an alkenyl group, a $C_{3-6}$ cycloalkyl group, a phenyl group substituted with a halogen atom or a divalent group of $-OCH_2{*}CH(CH_3)-$, $-SCH_2{*}CH_2-$or $-SCH_2{*}CH(CH_3)-$ which forms an oxazine or thiazine ring together with the nitrogen atom to which $R_1$ is attached and with X wherein X is C-Y;

$R_2$ is a hydrogen atom, a carboxy protecting group or a pharmaceutically acceptable metal or organic cation;

$R_3$ and $R_4$, which may be the same or different, are a hydrogen atom, a lower alkyl group, an acyl group or a nitrogen protecting group metabolizable in vivo;

Z is a hydrogen or halogen atom, an amino, hydroxy or methyl group; and

n is 1 to 3.

Field of the Invention

The present invention relates to novel quinolone carboxylic acid derivatives, and pharmaceutically acceptable salts thereof, substituted at 7-position of the quinolone or at 10-position of the pyridobenzoxazine which possess a broad spectrum of potent anti-bacterial activities; and to novel processes for preparing these compounds.

Description of the Prior Art

Quinolones such as enoxacin, norfloxcain, ofloxacin, ciprofloxacin, tosufloxacin and the like have been commercially available for sometime. However, most of these prior art materials are known to have relatively weak anti-bacterial potency especially against Gram-positive bacteria; and resistant microorganisms against these drugs have been found to exist.

Therefore, needs have existed for the development of new medicinal compounds which possess a broad scope of potent anti-bacterial activities and are effective against quinolone-resistant microorganisms including clinically important methicillin resistant Staphylococcus aureus(MRSA).

Summary of the Invention

Unexpectedly, the present inventors have discovered that certain quinolone carboxylic acid derivatives substituted at 7-position of the quinolone or at 10-position of the pyridobenzoxazine meet the above requirements.

Accordingly, the present invention primarily pertains to said novel quinolone compounds and pharmaceutically acceptable salts thereof; and novel processes for preparing these compounds.

Detailed Description of the Invention

The novel quinolone compounds of the present invention can be represented by the following formula-(I):

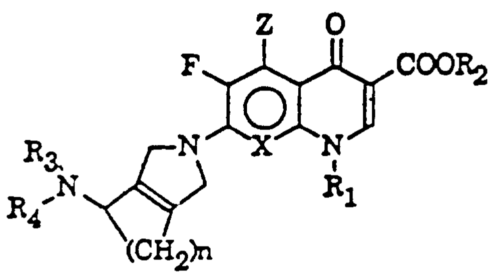

(I)

wherein:

| | |
|---|---|
| X is | a nitrogen atom or a C-Y group wherein Y is a hydrogen, fluorine, chlorine or bromine atom or a methoxy or methyl group; |
| $R_1$ is | a $C_{1-6}$ alkyl group optionally substituted with a halogen atom or a hydroxy radical, an alkenyl group, a $C_{3-6}$ cycloalkyl group, a phenyl group substituted with a halogen atom or a divalent group of $-OCH_2{}^*CH(CH_3)-$, $-SCH_2{}^*CH_2-$or $-SCH_2{}^*CH(CH_3)-$ which forms an oxazine or thiazine ring together with the nitrogen atom to which $R_1$ is attached and with X wherein X is C-Y; |
| $R_2$ is | a hydrogen atom, a carboxy protecting group or a pharmaceutically acceptable metal or organic cation; |
| $R_3$ and $R_4$, | which may be the same or different, are a hydrogen atom, a lower alkyl group, an acyl group or a nitrogen protecting group metabolizable in vivo; |
| Z is | a hydrogen or halogen atom, an amino, hydroxy or methyl group; and |
| n is | 1 to 3. |

The compounds of formula(I) encompass their acid or base addition salts and hydrates.

In the definition of $R_1$, said $C_{1-6}$ alkyl group is preferably a methyl, ethyl, n-propyl, isobutyl, t-butyl, n-pentyl or n-hexyl group, more preferably, an ethyl or t-butyl group;

said $C_{1-6}$ haloalkyl group is preferably a $C_{2-4}$ alkyl group substituted with a fluorine atom, e.g., 2-fluoroethyl group;

said $C_{1-6}$ hydroxyalkyl group is preferably a $C_{2-4}$ hydroxyalkyl group, e.g., 2-hydroxyethyl group;

said $C_{3-6}$ cycloalkyl group is preferably a cyclopropyl, cyclobutyl, cyclopentyl or cyclohexyl group, more preferably, a cyclopropyl group;

said alkenyl group is preferably a vinyl, isopropenyl, propenyl or isobutenyl group;

said phenyl group substituted with a halogen atom is preferably a phenyl group substituted with one or two fluorine atoms, more preferably, 4-fluorophenyl or 2,4-difluorophenyl group; and

the stereochemical configuration of the chiral atom of $-OCH_2{}^*CH(CH_3)-$ or $-SCH_2{}^*CH(CH_3)-$ group may be (R), (S) or a mixture thereof.

In the definition of $R_2$, said carboxy protecting group is an ester moiety which can be readily, e.g., hydrolyzed to produce a free carboxylic acid, e.g., a lower alkyl group such as methyl, ethyl, n-propyl or t-butyl group, a lower alkanoyloxy-lower alkyl group such as acetoxymethyl or pivaloyloxymethyl group, a lower alkoxycarbonyloxy-lower alkyl group such as methoxycarbonyloxymethyl or 1-ethoxycarbonyloxyethyl group, a lower alkoxymethyl group such as methoxymethyl group, a di(lower alkyl)amino-lower alkyl group such as 1-dimethylaminoethyl group, or a 4-methylene-5-methyl-1,3-dioxolene-2-one group; and

said pharmaceutically acceptable metal or organic cation is preferably a cation of alkaline metal or alkaline earth metal such as sodium, potassium, silver, calcium or magnesium cation or an organic cation such as tertiary or quarternary $C_{1-4}$ alkyl ammonium cation.

In the definition of $R_3$ and $R_4$, said lower alkyl group is preferably a methyl, ethyl, propyl or butyl group, more preferably, a methyl or ethyl group;

said acyl group is preferably an acetyl, propionyl or benzoyl group; and

said nitrogen protecting group metabolizable in vivo is preferably a formyl, alkoxycarbonyl, alkylcarbonylmethylene, alkoxycarbonylmethylene or 4-methylene-5-methyl-1,3-dioxolene-2-one group.

In the definition of Z, said halogen atom is preferably a fluorine atom.

In the definition of n, n is preferably 2 or 3.

The stereochemical configuration of azabicycloamine substituted at 7-position of the compounds of formula(I) may be(R), (S) or a mixture thereof.

Among the compounds of the present invention, preferred are: 7-[6-amino-3-azabicylo[3,3,0]oct-1(5)ene-3-yl]-1-cyclopropyl-6,8-difluoro-1,4-dihydro-4-oxoquinoline-3-carboxylic acid or its hydrochloride salt or its lactic acid salt, 1-cyclopropyl-6,8-difluoro-7-[6-methylamino-3-azabicyclo[3,3,0]oct-1(5)ene-3-yl]-1,4-dihydro-4-oxoquinoline-3-carboxylic acid, 7-[6-amino-3-azabicyclo [3,3,0]oct-1(5)ene-3-yl]-1-cyclopropyl-8-chloro-6-fluoro-1,4-dihydro-4-oxoquinoline-3-carboxylic acid or its hydrochloride salt or its lactic acid salt, 7-[6-methylamino-3-azabicyclo[3,3,0]oct-1(5)ene-3-yl]-8-chloro-1-cyclopropyl-6-fluoro-1,4-dihydro-4-oxoquinoline-3-carboxylic acid, 7-[6-amino-3-azabicyclo[3,3,0]oct-1(5)ene-3-yl]-8-bromo-1-cyclopropyl-6-fluoro-1,4-dihydro-4-oxoquinoline-3-carboxylic acid, 7-[6-amino-3-azabicyclo[3,3,0]oct-1(5)ene-3-yl]-1-cyclopropyl-6-fluoro-8-methoxy-1,4-dihydro-4-oxoquinoline-3-carboxylic acid, 1-cyclopropyl-6-fluoro-7-[6-methylamino-3-azabicyclo[3,3,0]oct-1(5)ene-3-yl]-1,4-dihydro-4-oxo-1,8-naphthyridine-3-carboxylic acid, 7-[6-amino-3-azabicyclo[3,3,0]oct-1(5)ene-3-yl]-6,8-difluoro-1-ethyl-1,4-dihydro-4-oxoquinoline-3-carboxylic acid, 7-[6-amino-3-azabicyclo[4,3,0]non-1(6)ene-3-yl]-1-cyclopropyl-6,8-difluoro-1,4-dihydro-4-oxoquinoline-3-carboxylic acid, 7-[6-amino-3-azabicyclo[3,3,0]oct-1(5)ene-3-yl]-5-amino-1-cyclopropyl-6,8-difluoro-1,4-dihydro-4-oxoquinoline-3-carboxylic acid, 7-[6-methylamino-3-azabicyclo[3,3,0]oct-1(5)ene-3-yl]-5-amino-1-cyclo-propyl-6,8-difluoro-1,4-dihydro-4-oxoquinoline-3-carboxylic acid, 7-[6-amino-3-azabicyclo[3,3,0]oct-1-(5)ene-3-yl]-1-[2,4-difluorophenyl-6,8-difluoro-1,4-dihydro-4-oxoquinoline-3-carboxylic acid, 7-[6-methylamino-3-azabicyclo[3,3,0]oct-1(5)ene-3-yl]-1-(2,4-difluorophenyl)-6,8-difluoro-1,4-dihydro-4-oxoquinoline-3-carboxylic acid, 7-[6-amino-3-azabicyclo[3,3,0]oct-1(5)ene-3-yl]-1-cyclopropyl-6-fluoro-1,4-dihydro-4-oxo-1,8-naphthyridine-3-carboxylic acid, 7-[6-methylamino-3-azabicyclo[3,3,0]oct-1(5)ene-3-yl]-1-cyclopropyl-6-fluoro-1,4-dihydro-4-oxo-1,8-naphthyridine-3-carboxylic acid, 9-fluoro-7-[6-amino-3-azabicyclo-[3,3,0]oct-1(5)ene-3-yl]-3-(S)-methyl-7-oxo-2,3-dihydro-7H-pyrido[1,2,3-de]-1,4-benzoxazine-6-carboxylic acid, 9-fluoro-7-[6-methylamino-3-azabicyclo[3,3,0] oct-1(5)ene-3-yl]-3-(S)methyl-7-oxo-2,3-dihydro-7H-pyrido[1,2,3-de]-1,4-benzoxazine-6-carboxylic acid, 7-[6-amino-3-azabicyclo[3,3,0] oct-1(5)ene-3-yl]-1-ethyl-6-fluoro-1,4-dihydro-4-oxoquinoline-3-carboxylic acid, 7-[6-methyl-amino-3-azabicyclo[3,3,0]oct-1(5)ene-3-yl]-1-ehtyl-6-fluoro-1,4-dihydro-4-oxoquinoline-3-carboxylic acid, 7-[6-amino-3-azabicyclo[3,3,0]oct-1(5)ene-3-yl]-1-ethyl-6-fluoro-1,4-dihydro-4-oxoquinoline-3-carboxylic acid, 7-[6-methylamino-3-azabicyclo[3,3,0]oct-1(5)ene-3-yl]-1-ethyl-6-fluoro-8-chloro-1,4-dihydro-4-oxoquinoline-3-carboxylic acid, 7-[6-amino-3-azabicyclo [3,3,0]oct-1(5)ene-3-yl-1-ethyl-5-amino-6,8-difluoro-1,4-dihydro-4-oxoquinoline-3-carboxylic acid, 7-[6-methylamino-3-azabicyclo [3,3,0]oct-1(5)ene-3-yl]-1-ethyl-5-amino-6,8-difluoro-1,4-dihydro-4-oxoquinoline-3-carboxylic acid, 7-[6-amino-3-azabicyclo[3,3,0] oct-1(5)ene-3-yl]-1-(t-butyl)-6,8-difluoro-1,4-dihydro-4-

oxoquinoline-3-carboxylic acid, 7-[6-methylamino-3-azabicyclo[3,3,0]oct-1(5)ene-3-yl]-1-(t-butyl)-6,8-difluoro-1,4-dihydro-4-oxoquinoline-3-carboxylic acid, 7-[6-amino-3-azabicyclo[3,3,0]oct-1(5)ene-3-yl]-1-(t-butyl)-6-fluoro-8-chloro-1,4-dihydro-4-oxoquinoline-3-carboxylic acid, 7-[6-methylamino-3-azabicyclo[3,3,0]oct-1(5)-ene-3-yl]-1-(t-butyl)-6-fluoro-8-chloro-1,4-dihydro-4-oxoquinoline-3-carboxylic acid, 7-[6-amino-3-azabicyclo-[3,3,0]oct-1(5)ene-3-yl)-1-(4-fluorophenyl)-6-fluoro-1,4-dihydro-4-oxoquinoline-3-carboxylic acid, 7-[6-amino-3-azabicyclo[3,3,0]oct-1(5)ene-3-yl]-1-(4-fluorophenyl)-6,8-difluoro-1,4-dihydro-4-oxoquinoline-3-carboxylic acid, 7-(6-methylamino-3-azabicyclo[3,3,0]oct-1(5)ene-3-yl]-1-(4-fluorophenyl)-6,8-difluoro-1,4-dihydro-4-oxoquinoline-3-carboxylic acid, 7-[6-amino-3-azabicyclo [3,3,0]oct-1(5)ene-3-yl]-1-ethyl-6-fluoro-1,4-dihydro-4-oxo-1,8-napthyridine-3-carboxylic acid, 7-[6-methylamino-3-azabicyclo[3,3,0] oct-1(5)ene-3-yl)-1-ethyl-6-fluoro-1,4-dihydro-4-oxo-1,8-naphthyridine-3-carboxylic acid, 7-[6-amino-3-azabicyclo[3,3,0]oct-1(5)ene-3-yl]-1(t-butyl)-6-fluoro-1,4-dihydro-4-oxoquinoline-3-carboxylic acid, 7-[6-methylamino-3-azabicyclo[3,3,0]oct-1-(5)ene-3-yl]-1-(t-butyl)-6-fluoro-1,4-dihydro-4-oxoquinoline-3-carboxylic acid, 7-[6-acetamino-3-azabicyclo-[3,3,0]oct-1(5)ene-3-yl]-1-cyclopropyl-6,8-difluoro-1,4-dihydro-4-oxoquinoline-3-carhoxylic acid and 7-[6-t-butoxycarbonylamino-3-azabicyclo[3,3,0]oct-1(5)ene-3-yl]-1-cyclopropyl-6,8-difluoro-1,4-dibydro-4-oxoquinoline-3-carboxylic acid methoxyethyl ester.

More preferred are: 7-[6-amino-3-azabicyclo[3,3,0]oct-1(5) ene-3-yl]-1-cyclopropyl-6,8-difluoro-1,4-dihydro-4-oxoquinoiine-3-carboxylic aicd or its hydrochloric acid salt or its lactic acid salt, 1-cyclopropyl-6,8-difluoro-7-[6-methylamino-3-azabicyclo[3,3,0] oct-1(5)ene-3-yl]-1,4-dihydro-4-oxoquinoline-3-carboxylic acid, 7-[6-amino-3-azabicyclo[3,3,0]oct-1(5)ene-3-yl]-1-cyclopropyl-8-chloro-6-fluoro-1,4-dihydro-4-oxoquinoline-3-carboyxlic acid or its hydrochloric acid salt or its lactic acid salt, 7-[6-methylamino-3-azabicyclo[3,3,0]oct-1-(5)ene-3-yl]-8-chloro-1-cyclopropyl-6-fluoro-1,4-dihydro-4-oxoquinoline-3-carboxylic acid, 7-[6-amino-3-azabicyclo [3,3,0]oct-1(5)ene-3-yl]-1-cyclopropyl-6-fluoro-8-methoxy-1,4-dihydro-4-oxoquinoline-3-carboxylic acid, 7-[6-methylamino-3-azabicyclo[3,3,0]oct-1(5)ene-3-yl]-1-cyclopropyl-6-fluoro-1,4-dihydro-4-oxo-1,8-naphthyridine-3-carboxylic acid, 7-[6-amino-3-azabicyclo [3,3,0]oct-1(5)ene-3-yl]-5-amino-1-cyclopropyl-6,8-difluoro-1,4-dihydro-4-oxoquinoline-3-carboxylic acid, 7-[6-amino-3-azabicyclo [3,3,0]oct-1(5)ene-3-yl]-1-cyclopropyl-6-fluoro-1,4-dihydro-4-oxo-1,8-naphthyridine-3-carboxylic acid, 9-fluoro-10-[6-amino-3-azabicyclo[3,3,0]oct-1(5)ene-3-yl]-3-(S)-methyl-7-oxo-2,3-dihydro-7H-pyrido[1,2,3,-de]-1,4-benzoxazine-6-carboxylic acid, 9-fluoro-10-[6-methylamino-3-azabicyclo[3,3,0]oct-1(5)ene-3-yl]-3-(S)methyl-7-oxo-2,3-dihydro-7H-pyrido[1,2,3-de]-1,4-benzoxazine-6-carboxylic acid, 7-[6-amino-3-azabicylo[3,3,0]oct-1(5)ene-3-yl]-1-(t-butyl)-6-fluoro-1,4-dihydro-4-oxoquinoline-3-carboxylic acid, 7-[6-t-butoxycarbonylamino-3-azabicyclo[3,3,0]oct-1(5)ene-3-yl]-1-cyclopropyl-6,8-difluoro-1,4-dihydro-4-oxoquinoline-3-carboxylic acid.

The quinolone compounds of the present invention represented by formula(I) and pharmaceutically acceptable salts thereof can be prepared as follows.

Process A

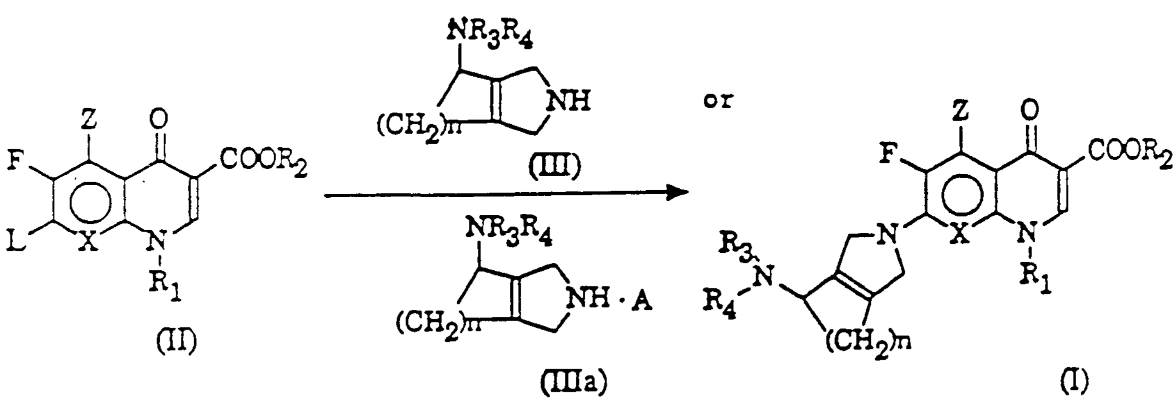

(II) (III) or (IIIa) (I)

wherein:

| | |
|---|---|
| $R_1$, $R_3$, $R_4$, X, Z and n | are the same as defined previously; |
| $R_2$ is | a hydrogen atom or a carboxy protecting group; |
| L is | a leaving group which is a fluorine, chlorine or bromine atom, a methanesulfonyl group or a para-toluenesulfonyl group; and, |
| A is | a hydrochloric, bromic, sulfuric or trifluoroacetic acid. |

In accordance with the above Process A, the quinolone compounds of formula(I) wherein $R_2$ is a hydrogen atom or a carboxy protecting group(hereinafter referred to as the compounds of formula (Ia)) can be prepared by reacting the compounds of formula(II) with the compounds of formula(III) or (IIIa) in the presence of a base.

The compounds of formula(II) are well-known in the art and the compounds of formula(III) are novel and can be prepared in accordance with the methods described below.

The above reaction can be carried out in the presence of an inert solvent such as methanol, ethanol, isopropanol, acetonitrile, pyridine, dimethylformamide, dimethylsulfoxide, dioxane, etc. or a mixture thereof at a temperature ranging from 10 and 200 °C preferably 50 and 120 °C for 10 min to 24 hrs.

A base such as triethylamine, diisopropylethylamine, pyridine, 1,8-diazabicyclo[5,4,0]undec-7-ene or alkaline metal or alkaline earth metal carbonate may be used to neutralize the acids produced in the above reaction.

The compounds of formula(Ia) obtained from Process A wherein $R_2$ is a hydrogen atom(hereinafter referred to as the compounds of formula(Ia-1)) can be converted to the compounds of formula(Ia) wherein $R_2$ is a carboxy protecting group(hereinafter referred to as the compounds of formula(Ia-2)) in accordance with the following Process B.

Process B

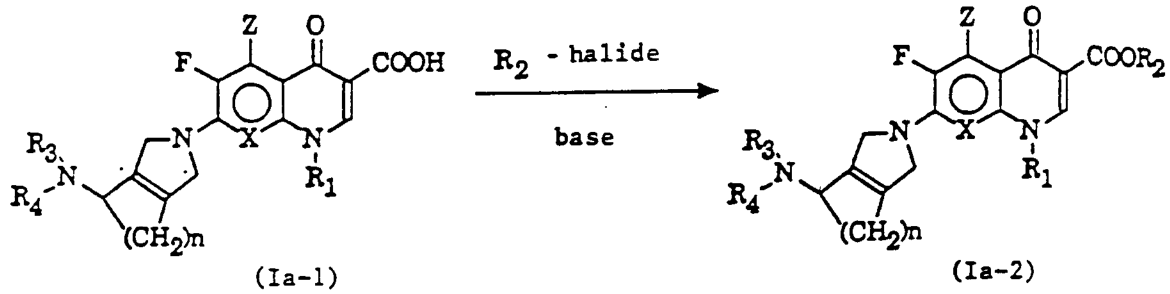


(Ia-1) (Ia-2)

wherein:

| | |
|---|---|
| $R_1$, $R_3$, $R_4$, Z, X and n | are the same as defined previously; |
| Hal is | a chlorine, bromine or iodine atom; and |
| $R_2$ is | a carboxy protecting group which is a lower alkyl group such as methyl, ethyl, n-propyl or t-butyl group, a lower alkanoyloxy-lower alkyl group such as acetoxymethyl or pivaloyloxymethyl group, a lower alkoxycarbonyloxy-lower alkyl group such as methoxycarbonyloxymethyl or 1-ethoxycarbonyloxyethyl group, a lower alkoxymethyl group such as methoxymethyl group, a di(lower alkyl) amino-lower alkyl group such as dimethylaminoethyl group or a 4-methylene-5-methyl-1,3-dioxolene-2-one group. |

In accordancce with the above Process B, the compounds of formula(Ia-2) can be prepared by reacting the compounds of formula (1a-1) with the compounds of $R_2$-Hal in an inert solvent such as haloalkane or dimethylformamide in the presence of a base such as diisopropylethylamine.

The compounds of formula(Ia-1) obtained from Process A can be converted to the compounds of formula(Ia) wherein $R_2$ is a pharmaceutically acceptable metal or organic cation(hereinafter referred to as the compounds of formula(Ia-3)) in accordance with the following Process C.

Process C

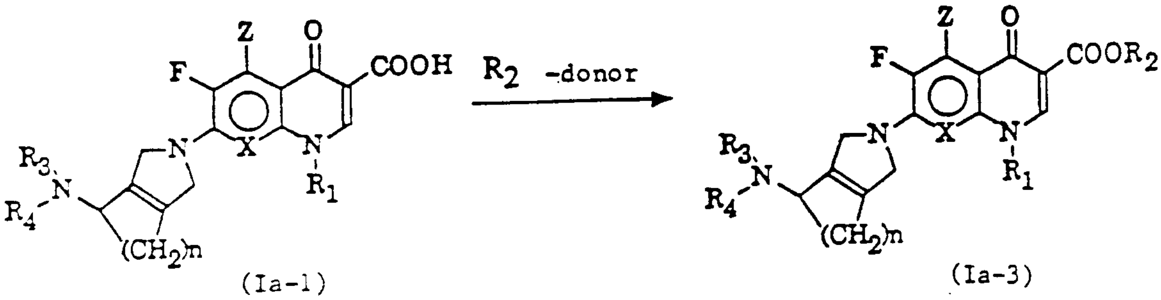


(Ia-1) (Ia-3)

wherein:

$R_1$, $R_3$, $R_4$, Z, X and n are the same as defined previously; and

$R_2$ is a pharmaceutically acceptable metal or organic cation.

In accordance with the above Process C, the compounds of formula(Ia-3,) can be prepared by dissolving the compounds of formula (Ia-1) in a lower alcohol or haloalkane solvent; adding thereto an $R_2$-donor in water or a lower alcohol; and collecting the produced precipitates or removing the solvent.

Said $R_2$-donor used in the above reaction may be sodium hydroxide, potassium hydroxide, calcium hydroxide, magnesium hydroxide, silver nitrate or organic cation compounds.

The compounds of formula(Ia-3) may further encompass, their hydrates.

The compounds of formula(Ia) obtained from Process A wherein $R_4$ is a hydrogen atom(hereinafter referred to as the compounds of formula(Ia-4)) can be converted to the compounds of formula(Ia) wherein $R_4$ is the same as defined in the formula(Ia), excepting a hydrogen atom(hereinafter referred to as the compounds of formula(Ia-5)) by using the following Process D.

Process D

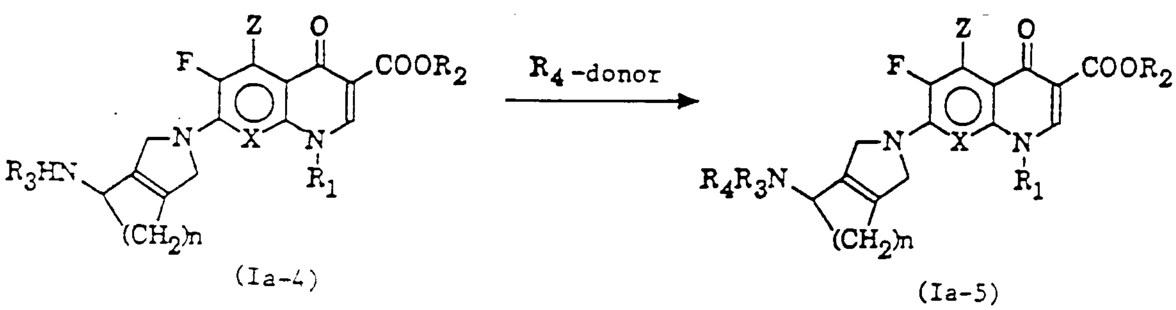


(Ia-4) (Ia-5)

wherein:

$R_1$, $R_2$, $R_3$, Z, X and n are the same as defined in the formula (Ia); and

$R_4$ is the same as defined in the formula(Ia), except a hydrogen atom.

In accordance with the above Process D, the compounds of formula(Ia-5) can be prepared by reacting the compounds of formula (Ia-4) with an $R_4$-donor of formula $R_4$-Hal(wherein Hal is a chlorine, bromine or iodine atom) in the presence of a base such as triethylamine, diisopropylethylamine, 1,8-diazabicyclo[5,4,0]-undec-7-ene, sodium hydride, potassium hydride, calcium hydride or alkaline metal carbonate.

The above process is advantageous especially when $R_3$ is a hydrogen atom or an alkyl group and $R_4$ is a nitrogen protecting group metabolizable in vivo.

If $R_4$ is a formyl group, the compound of formula(Ia-4) is reacted with acetic anhydride or sodium acetate in formic acid; and if $R_4$ is an acyl group, with an acid anhydride or halide having two to four carbon atoms in the presence of a base such as triethylamine or pyridine.

The compounds of formula(Ia) may be converted to acid addition salts thereof with an inorganic or organic acid such as hydrochloric, sulfuric, phosphoric, nitric, methanesulfonic, acetic, formic, propionic,

lactic, maleic, malonic, fumaric, tartaric, citric, ascorbic, glutamic, paratoluenesulfonic acid, etc; and to their hydrates.

The above conversion reaction is carried out by dissolving the compounds of formula(Ia) in a solvent, e.g., a lower alcohol such as methanol or ethanol, or a haloalkane such as chloroform, dichloromethane or dichloroethane; adding an aqueous or alcoholic solution of a selected organic or inorganic acid; and collecting the precipitates produced therefrom or removing the solvent.

The present invention further encompasses a pharmaceutical composition comprising one or more compounds of formula(I) and non-toxic inert excipients.

The compounds of the present invention can be administered locally, orally, parenterally or rectally, preferably intravenously or intramuscularly. In general, it has been shown advantageous to administer the compounds of the present invention in an amout of about 0.5 to 500, preferably 1 to 100mg/kg of body weight per day in single or divided doses. A dosage ranging from about 1 to 200mg/kg of body weight, particularly 1 to 50mg/kg of body weight is preferred. However, it will be understood that the amount of the compound actually administered say be adjusted in the light of the relevant circumstances including the form of formulation, the chosen route of administration, the age, weight and response of the individual patient, and the severity of the patient's symptoms.

One or more compounds of the present invention may be either administered as such or formulated for administration by mixing therewith non-toxic, inert pharmaceutically acceptable expients such as solid, semi-solid or liquid diluent, filler and carrier. Examples of such formulation are in the form of: a tablet, lozenge, capsule, granule, suppository, solution, suspension, emulsion, paste, ointment, cream, lotion, powder, spray and the like.

In case of tablet, lozenge, capsule and granule, the active compounds of the present invention may be combined with conventional expients, e.g., fillers and extenders such as starch, lactose, sucrose, glucose mannitol and the like, binders such as carboxymethyl cellulose, alginate, gelatine, polyvinylpyrolidone and the like; disintegrants such as calcium carbonate, sodium bicarbonate and the like; absorption accelerants such as quarternary ammonium compound and the like; wetting agents such as cetyl alcohol, glycerine monostearate and the like; adsorbents such as kaoline, bentonite and the like; lubricants such as talc, calcium stearate, magnesium stearate, solid polyethylene glycol and the like; or mixtures thereof. The tablet, lozenge, capsule, pill and granule may be coated with conventional coating materials including any opacifier.

The suppository may contain conventional soluble or insoluble expients, e.g., polyethylene glycol, fat, polymeric ester or a mixture thereof in addition to the active compound.

The ointment, paste, cream and the like may contain conventional expients, e.g., animal or vegetable fat, wax, paraffin, starch, cellulose derivatives, polyethylene glycol, bentonite, talc, zine oxide or a mixture thereof in addition to the active compounds.

The solution or emulsion for oral administration may contain conventional expients such as solvents, solubilizers and emulsifiers, e.g., water, ethyl alcohol, benzyl benzoate, propylene glycol, cotton seed oil, pinutts oil, corn oil, olive oil, fatty esters such as glycerine, polyethylene glycol or sorbitan, or a mixture thereof in addition to the active compounds.

The solution, suspension or emulsion for parenteral administration may contain a sterilized isometric solution or emulsion. In particular, the suspension may contain the expients, e.g., liquid diluent or suspending agent such as water, ethyl alcohol or propylene glycol.

The formulations may comprise about 0.1 to 99.5% by weight, preferably 0.5 to 95% by weight of the active compounds of the present invention; and may further contain conventionally accepted amounts of dyes, preservatives, sweetners and additives.

The present invention also relates to unsaturated azabicycloamine compounds and processes for the preparation thereof, and, more specifically, to unsaturated azabicycloamine compounds useful as an intermediate for preparing quinolone carboxylic acids, cephalosporin compounds, and other related anti-bacterial compounds; and to novel processes for preparing these compounds and the salts thereof.

The present unsaturated azabicycloamine compounds which do not have stereoisomers relating to the form of their rings but have a planar molecular shape are useful for preparing various different antibacterial compounds such as cephalosporin and quinolone carboxylic acid derivatives with improved activities.

Accordingly, the present invention also pertains to providing novel azabicycloamine compounds useful as intermediate compounds for preparing such diverse and effective antibacterial compounds; and novel processes for the preparation thereof.

The present invention relates to the azabicycloamine compounds, and their salts represented by the formula(I):

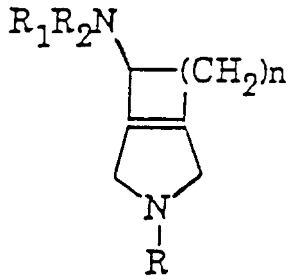

(I)

wherein:

R is a hydrogen atom or an amine protecting group;

$R_1$ and $R_2$, which may be the same or different, are hydrogen, optionally substituted lower alkyl, alkanoyl, optionally substituted benzoyl or naphthoyl, optionally substituted alkoxycarbonyl, or optionally substituted benzyloxycarbonyl; and

n is an integer from 1 to 4.

The amine protecting group of R in formula (I) may be benzenesulfonyl, paratoluenesulfonyl, methanesulfonyl, $C_{1-4}$-alkoxycarbonyl, benzyloxycarbonyl, paranitrobenzyloxycarbonyl, 2,2,2-trichlorethoxycarbonyl, $C_{1-20}$-alkanoyl, or optionally substituted benzoyl or naphthoyl; $R_1$ or $R_2$ may be optionally substituted lower alkyl, such as $C_{1-7}$-, preferably $C_{1-4}$-alkyl, especially methyl or ethyl, alkanoyl, preferably $C_{1-18}$-alkanoyl, including formyl, acetyl, propionyl, butanoyl, pentanoyl, hexanoyl, heptanoyl, octanoyl, decanoyl, undecanoyl, palmitoyl, oleoyl, stearoyl and cyclohexanoyl, optionally substituted benzoyl or naphthoyl, optionally substituted alkoxycarbonyl, preferably $C_{1-4}$-alkoxycarbonyl, such as ethoxycarbonyl, and 2,2,2-trichloroethoxycarbonyl, or optionally substituted benzyloxycarbonyl, such as paranitrobenzyloxycarbonyl.

Another aspect of the present invention relates to an acid addition salt of the compound of formula (I) wherein at least one nitrogen atom is basic nitrogen, which may include the salts of an inorganic acid such as hydrochloric, hydrobromic, phosphoric, or sulfuric acid, and of an organic acid such as formic, acetic propionic, methane sulfonic, trichloroacetic, trifluoroacetic, lactic, maleic, malonic, fumaric, citric, tartaric, paratoluene sulfonic, benzene sulfonic, palmitic, oleic or stearic acid.

The process for preparing the compound of formula (Ia-1) may be summarized by the following Scheme(A):

Scheme(A)

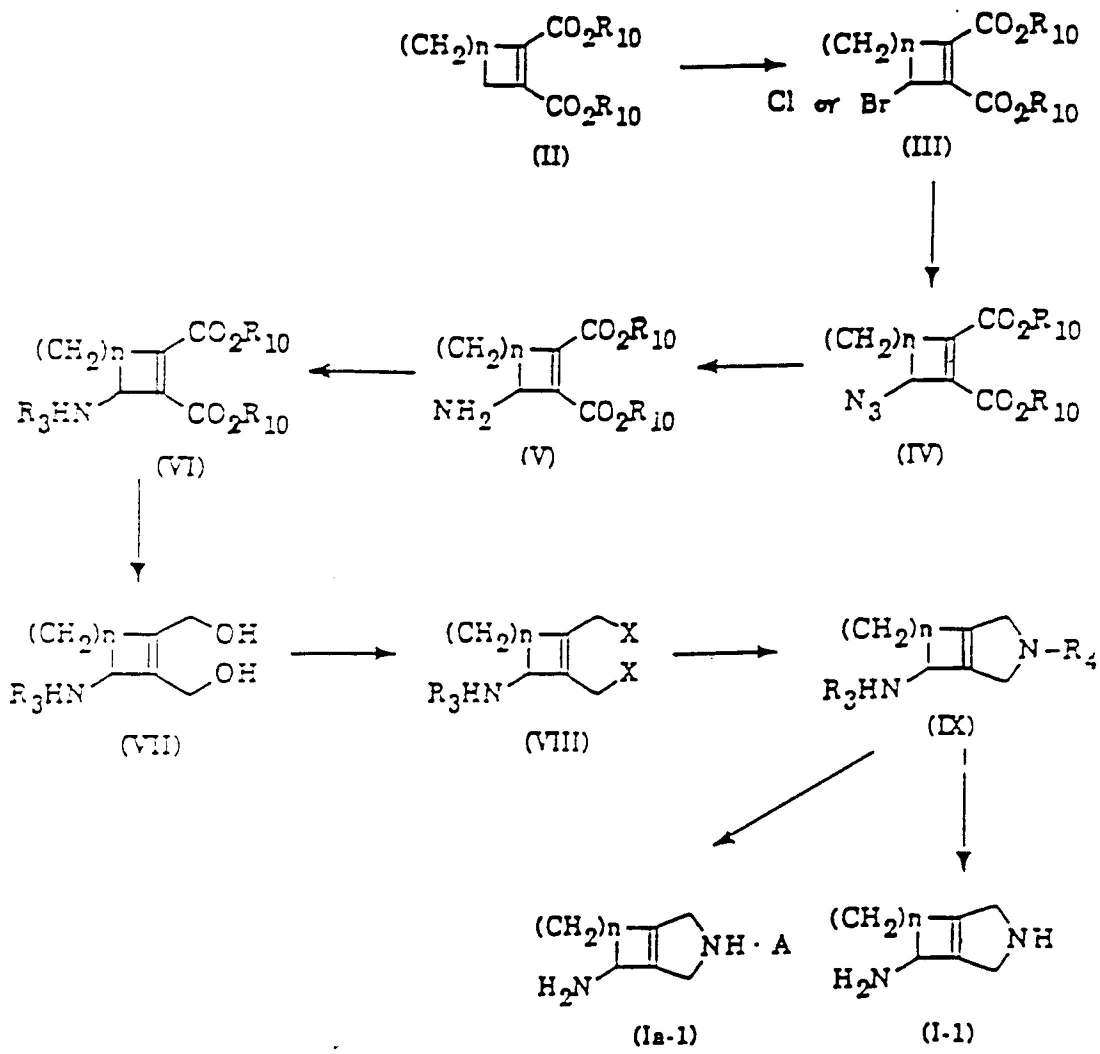

wherein:

| | |
|---|---|
| n is | an integer from 1 to 4; |
| $R_{10}$ is | a $C_{1-4}$-alkyl group; |
| $R_3$ an | amine protecting group, such as e.g. methoxycarbonyl, ethoxycarbonyl, t-butoxycarbonyl, 2,2,2-trichloroethorycarbonyl, benzyloxycarbonyl, or paranitrobenzyloxycarbonyl; |
| $R_4$ has | the same definition as R in formula (I), especially benzenesulfonyl, paratoluenesulfonyl, methanesulfonyl, or $C_{1-4}$-alkoxycarbonyl; |
| X is | iodine, bromine or chlorine, or a O-mesyl or O-tosyl grou; and |
| A is | hydrochloric, hydrobromic, sulfonic or trifluoroacetic acid. |

The compound of formula (IV) may be prepared by reacting sodium azide with the compound of formula (III) which may be prepared by heating the compound of formula (II) with N-bromo(or chloro) succinimide in the presence of a free radical-generating agent such as benzoyl peroxide or AIBN (aziisobutyronitrile).

The compound of formula (V) is prepared by reacting the compound of formula (IV) with triphenylphosphine in the presence of a solvent of tetrahydrofuran and water; and, thereafter, the compound of formula (VI) is prepared by reacting the compound of formula (V) with an amine protecting agent such as di-t-butyl dicarbonate or methyl(or ethyl-, trichloromethyl-, benzyl- or paranitrobenzyl-) chloroformate in the

presence of base such as pyridine, triethylamine, sodium hydride, sodium hydroxide, or potassium carbonate.

The compound of formula (VI) is reduced with an ester reducing agent such as diisobutylaluminium hydride to obtain the alcohol of formula (VII), which is subsequently reacted with a halogenation agent such as phosphorous trichloride, phosphorous trifluoride, thionylchloride or oxalkyl chloride, or reacted with triphenylphosphine and carbon tetrachloride or carbon tetrabromide.

The compound of formula (IX) can be prepared by reacting the compound of formula (VIII) with an $R_4$ donating agent such as paratoluenesulfonamide, benzenesulfonamide, acetamide, benzamide, methanesulfonamide and urethane in the presence of a base such as sodium hydride, triethylamine or potassium carbonate.

The compound of formula (IX) is heated in hydrochloric acid, sulfuric acid, hydrobromic acid or trifluoroacetic acid to obtain the compound of formula (Ia-1); and may also be used for preparing the compound of formula (I-1) by removing the amine protecting group in the presence of a base such as sodium hydroxide or sodium ammonia.

The compound of formula (I) wherein R and $R_1$ are hydrogen and $R_2$ is lower alkyl (compound I-2, Ia-2) can be prepared in accordance with the following Scheme (B):

Scheme(B)

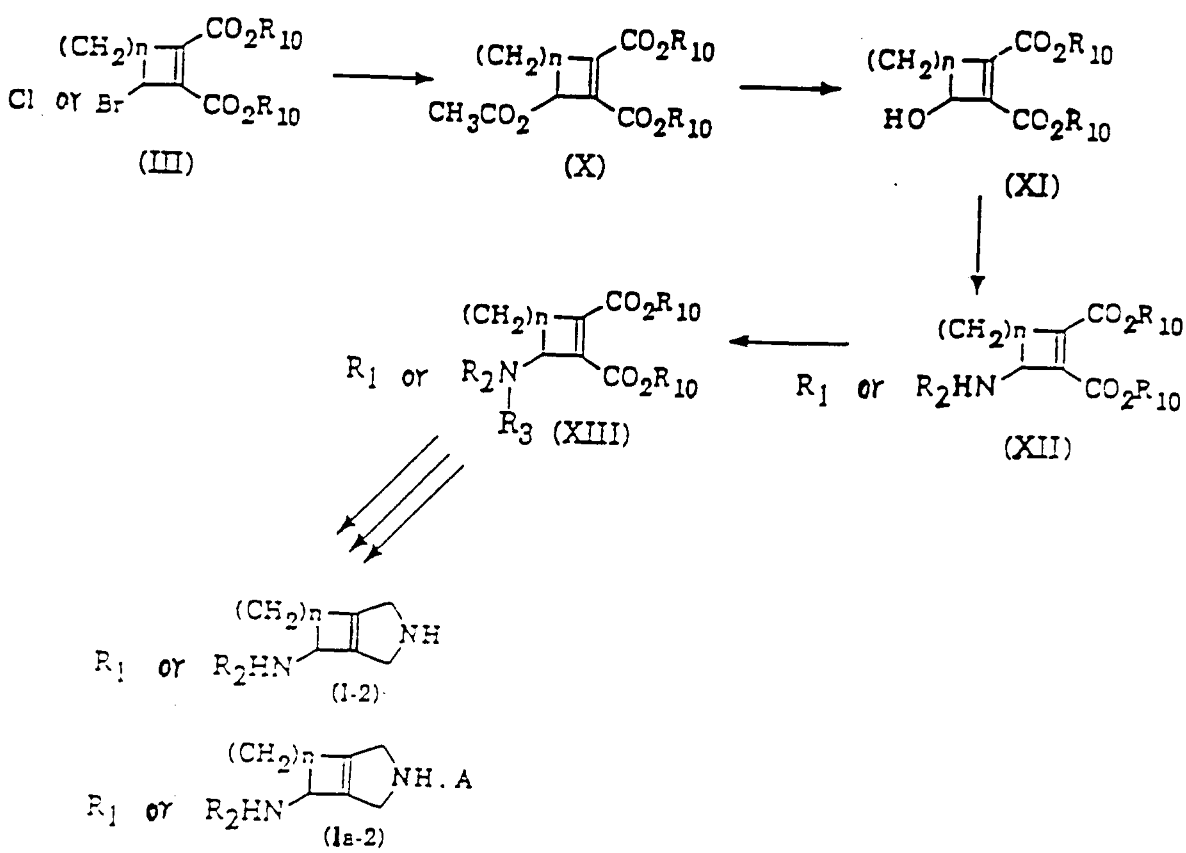

(III) (X) (XI) (XII) (XIII) (I-2) (Ia-2)

wherein:

n, $R_{10}$, $R_2$, $R_3$ and A    have the same meanings as defined previously.

In Scheme(B), a compound of formula(X) is obtained by reacting the compound of formula(III) with sodium acetate or silver acetate, and a compound of formula(X) is subsequently deacetylated to give the compound of formula(XI).

The compound of formula(XI) is tosylated or mesylated, which subsequently is either reacted with a $R_2$ donating amine compound or undergoes a Mitsunobu reaction so as to produce a compound of formula-(XII).

Protecting the nitrogen atom of formula(XII) with an amine protecting agent by employing the same method as used for the preparation of compound of formula(VI), the compound of formula(I-2) or (Ia-2) is prepared by repeating the process of preparing the compound of the formula(I-1) or (Ia-1).

Further, the compound of formula(I) wherein R and $R_1$ are hydrogen and $R_2$ is a lower alkyl group may be prepared by employing the method of Scheme (C) given below:

Scheme(C)

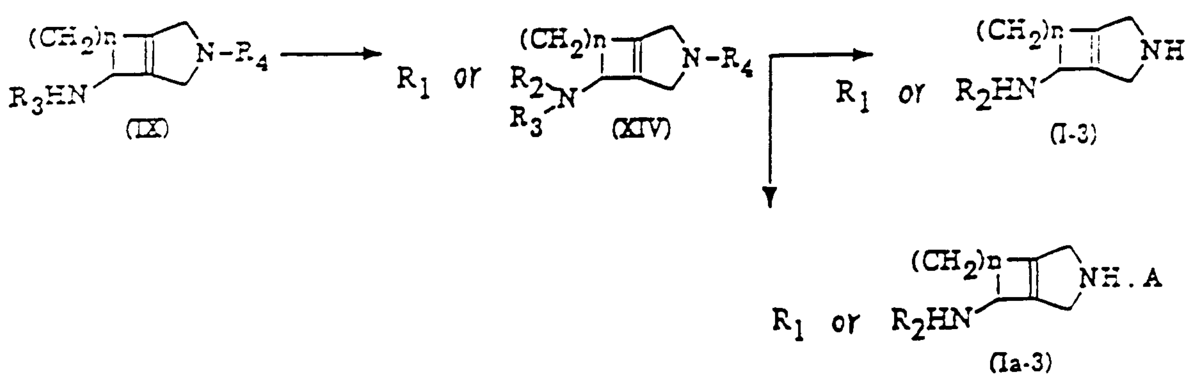

(IX) R1 or (XIV) R1 or (I-3) R1 or (Ia-3)

wherein:

n, $R_2$, $R_3$, $R_4$ and A have the same meanings as defined previously.

In Scheme(c), the compound of formula(IX) is reacted with an alkylhalide in the presence of a strong base such as sodium hydride to obtain the compound of formula(XIV), from which $R_3$ and $R_4$, the amine protecting groups are removed to produce the compound of formula(I-3) or (Ia-3).

Further, the compound of formula(I) wherein $R_1$ is a methyl group may be prepared by reducing the compound of formula (XV) in the presence of aluminium lithium hydride to obtain the compound of formula-(XVI) and removing $R_6$ therefrom as shown in the following Scheme(D):

Scheme(D)

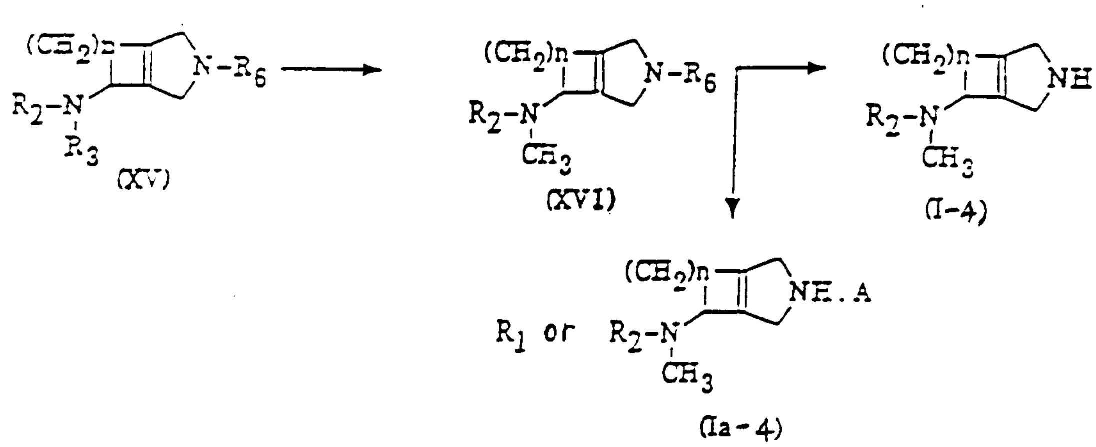

(XV) (XVI) (I-4) R1 or (Ia-4)

wherein:

n, A, $R_2$ and $R_3$ have the same meanings as previously defined; and

$R_6$ is a (para)toluenesulfonyl or methanesulfonyl group.

More specifically, the compound of formula(I), wherein one of $R_1$ and $R_2$ is a methyl group and the other is a hydrogen atom, can be prepared by employing the following Scheme(E):

Scheme(E)

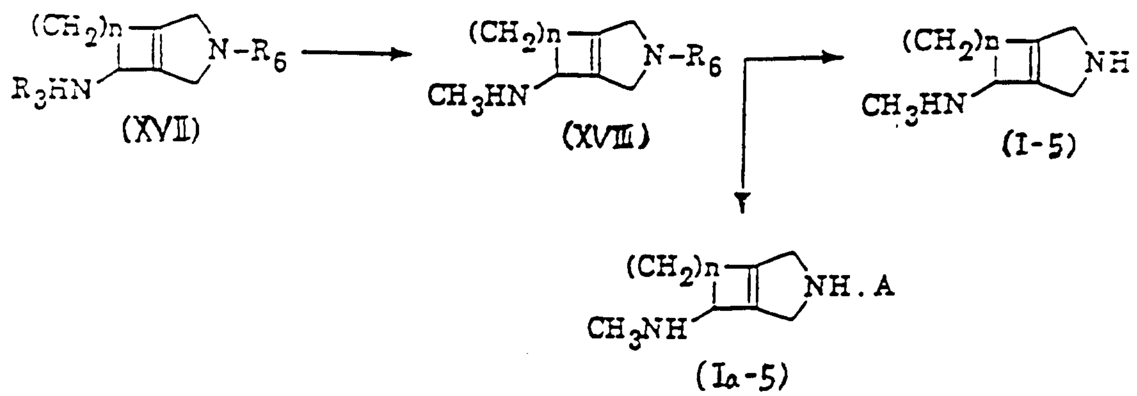

(XVII) (XVIII) (I-5) (Ia-5)

wherein:

n, A, $R_3$ and $R_6$ have the same meanings as previously defined; and

$R_6$ is a (para)toluenesulfonyl or methanesulfonyl group.

The compound of the formula(I-5) or (Ia-5) can be obtained by reducing $R_3$ in the compound of formula (XVII) to a methyl group in the presence of lithium aluminium hydride to give the compound of formula-(XVIII) and by way of removing $R_6$ therefrom.

The following examples are intended to further illustrate the synthesis of the special intermediates:

Example 1: Synthesis of 3-bromo-1-cyclopentene-1,2-dicarboxylic acid dimethylester

To a solution of 18g of 1-cyclopentene-1,2-dicarboxylic acid dimethylester dissolved in 180mℓ of carbon tetrachloride were added 17.4g of N-bromosuccinimide(NBS) and 1.6g of $\alpha,\alpha$-azobis (isobutyronitrile)(AIBN); and the resultant mixture was refluxed for 5 hours. After filtering the reaction mixture, the filtrate was concentrated under a reduced pressure and purified by fractionating on the silica gel column chromatography to obtain 18.8g of the title compound in yellow oil(yield 73%).

MS m/z(rel. int, %): 263($M^+$, 43), 231(23), 183(100), 151(63)

$^1$H-HMR($CDCl_3$, $\delta$ ppm): 2.45(1H, m), 2.57(1H, m), 2.7 (1H, m), 3.05(1H, m), 3.73(6H, s), 5.25(1H, m)

Example 2: Synthesis of 3-azido-1-cyclopentene-1,2-dicarboxylic acid dimethylester

To a solution of 18g of 3-bromo-1-cyclopentene-1,2-dicarboxylic acid dimethyl ester dissolved in 200mℓ of carbon tetrachloride was added a solution of 15.6g of sodium azide dissolved in 65mℓ of water; and the resultant solution was stirred for 16 hours. The reaction solution was refluxed for 1 hour and cooled; and the organic layer was separated. After the remaining aqueous layer was extracted twice with carbon tetrachloride, the organic layers were combined, dried over anhydrous magnesium sulfate and concentrated under a reduced pressure to obtain 13. 4g of the title compound in yellow oil(yield 92%).

MS m/s(rel. int, %): 226($M^+$, 3.3), 187(67), 151(100), 138(45), 106(30), 93(27), 68(27)

$^1$H-NMR($CDCl_3$, $\delta$ ppm): 2.0(1H, m), 2.4(1H, m), 2.67(1H, m) 2.9(1H, m), 3.8(6H, s), 4.77(1H, m)

Example 3: Synthesis of 3-amino-1-cyclopentene-1,2-dicarboxylic acid dimethylester

To a solution of 13.4g of 3-azido-1-cyclopentene-1,2-dicarboxylic acid dimethyl ester dissolved in 135mℓ of tetrahydro furan was added 15.5g of triphenylphosphine; and the resultant mixture was stirred for 16 hours under nitrogen gas.

After adding 1.6mℓ of water, the reaction mixture was stirred for 5 hours at a room temperature and concentrated under a reduced pressure; and the residue was dissolved in 250mℓ of dichloromethane. The reaction solution was extracted with 350mℓ of 2N hydrochloric acid, and, then, the aqueous layer was separated and neutralized with 15% aqueous NaOH solution.

The aqueous solution was extracted with 300mℓ of dichloromethane, dried over anhydrous magnesium sulfate and concentrated under a reduced pressure to obtain 5.9g of the title compound in pale yellow oil-(yield 50%).

MS m/z(rel. int, %); 199($M^+$, 4), 184(3.5), 152(15), 140 (100), 108(28), 80(55)

$^1$H-NMR($CDCl_3$, δ ppm): 1.6(2H, m), 2.4(1H, m), 2.6(1H, m), 2.75(1H, m), 3.7(1H, m), 3.73(3H, s), 3.75(3H, s), 4.2(1H, m)

Example 4: Synthesis of 3-(N-t-butoxycarbonyl)amino-1-cyclopentene-1,2-dicarboxylic acid dimethylester

To a solution of 5.9g of 3-amino-1-cyclpentene-1,2-dicarboxylic acid dimethylester dissolved in 80mℓ of methanol was added 7.1g of di-t-buthyl dicarbonate, and the resultant mixture was strirred for 16 hours at room temperature. The reaction mixture was concentrated under the reduced pressure and fractionated on silica gel column chromatography to obtain 8.42g of the title compound in white precipitate(yield 95%).

MS m/z(rel. int, %): 300($M^+$, 28), 244(88), 200(38), 183 (49), 167(53), 140(46), 57(100)

$^1$H-NMR($CDCl_3$, δ ppm): 1.4(9H, s), 1.73(1H, m), 2.8~2.4 (3H, m), 3.74(3H, s), 3.76(3H, s), 4.76(1H, br.s), 5.03(1H, br.s)

Example 5: Synthesis of 3-(N-t-butoxycarbonyl)amino-1,2-bis (hydroxymethyl)-1-cyclopentene

A solution of 18g of diisobutylaluminium hydride dissolved in 100mℓ of anhydrous tetrahydrofuran was cooled to -78°C and, then, a solution of 9.4g of 3-amino(N-t-butoxycarbonyl)amino-1-cyclopentene-1,2-dicarboxylic acid dimethyl ester dissolved in 90mℓ of tetrahydrofuran was added dropwise thereto for 1 hour. The resultant solution was stirred for 2 hours; and 9mℓ of methanol and 3mℓ of water were added carefully thereto.

The reaction mixture was extracted with 50% solution of methanol and dichloromethane(1:1, v/v) and concentrated under a reduced pressure to obtain 3.03g of the title compound in pale yellow oil(yield 40%).

MS m/z(rel. int, %): 243($M^+$)

$^1$H-NMR($CDCl_3$, δ ppm): 1.39(9H, s), 1.45(1H, m), 2.5~2.25 (3H, m), 4.03(1H, m), 4.2(4H, m), 4.68(1H, m)

Example 6: Synthesis of 3-(N-t-butoxycarbonyl)amino-1,2-bis (bromomethyl)-1-cyclopentene

To a solution of 3.03g of 3-amino(N-t-butoxycarbonyl)amino-1,2-bis(hydroxymethyl)-1-cyclopentene and 9.94g of carbon tetrabromide dissolved in 30mℓ of dichloromethane was added dropwise for 10 minutes a solution of 9.8g of triphenylphosphine dissolved in 20mℓ of dichloromethane, in a cold water bath. The resultant solution was stirred for 30 minutes in a cold water bath and concentrated under the reduced pressure; and the residue was dissolved in ethyl-ether, which was filtered for the removal of undissolved solids. The filtrate was concentrated under a reduced pressure and purified by fractionating on the silica gel column chromatography to obtain 1.85g of the title compound in white precipitate(yield 40%).

m.p. : 104° ~ 106°

MS m/z(rel. int, %): 369($M^+$), 312(50), 253(22), 234(90), 188(15), 152(100), 108(27), 91(23), 57-(55)

$^1$H-NMR($CDCl_3$, δ ppm): 1.58(9H, s), 1.65(1H, m), 2.35~2.65 (3H, m), 4.03(4H, m), 4.61(1H, br.s), 4.88(1H, br.s)

Example 7: Synthesis of 3-(N-t-butoxycarbonyl)amino-1,2-bis (chloromethyl)-1-cyclopentene

To a solution of 3.03g of 3-amino(N-t-butoxycarbonyl)-1,2-bis(hydroxymethyl)-1-cyclopentene dissolved in 30mℓ of carbon tetrachloride was added 6.86g of triphenylphosphine; and the resultant mixture was refluxed for 3 hours. The reaction mixture was concentrated under a reduced pressure and fractionated on the silica gel column chromatography to obtain 1.21g of the title compound in white precipitate(yield 35%).

m.p. : 85° ~ 88°

MS m/z(rel. int, %): 377($M^+$, 5), 222(85), 187(70), 163(50), 152(95), 127(27), 91(45), 57(100)

$^1$H-NMR($CDCl_3$, δ ppm): 1.39(9H, s), 1.6(1H, m), 2.4~2.6 (3H, m), 4.1(4H, m), 4.5(1H, br.s), 4.8(1H, br.s)

Example 8: Synthesis of 6-(N-t-butoxycarbonyl)amino-3-(paratoluenesulfonyl)-3-azabicyclo[3,3,0]oct-1(5)ene

To a solution of 1.85g of 3-amino(N-t-butoxycarbonyl) amino-1,2-bis(bromomethyl)-1-cyclopentene and 943mg of paratoluene sulfonamide dissolved in 20mℓ of dimethylformamide was added 500mg of sodium hydride(55~60%) in a cold water bath. The resultant mixture was stirred for 5 hours; and 60mℓ of water was added thereto. The reaction solution was, then extracted with dichloromethane, concentrated under a reduced pressure and purified by fractionating on the silica gel column chromatography to obtain 950mg of the title compound in white precipitate(yield 50%).

m.p. : 159° ~ 160°C

MS m/z(rel. int, %): 378($M^+$, 0.5), 321(18), 260(95), 234 (20), 167(100), 155(17), 106(93), 91 (27), 80(18), 57(17)

$^1$H-NMR($CDCl_3$, $\delta$ ppm): 1.5(9H, s), 1.88(1H, m), 2.09 (1H, m), 2.20(1H, m), 2.44(3H, s), 2.69(1H, s), 3.99(4H, br.s), 4.45 (1H, br.m), 4.56(1H, br.s), 7.31(2H, d, J=8Hz), 7.73(2H, d, J=8Hz)

Example 9: Synthesis of 6-amino-3-azabicyclo[3,3,0]oct-1(5)ene dihydrobromide

To a solution of 760mg of 6-amino(N-t-butoxycarbonyl)amino-3-(paratoluenesulfonyl)-3-azabicyclooct-1-(5)ene dissolved in 2.6mℓ of 30% aqueous hydrobromide was added 430mg of phenol. The resultant mixture was refluxed for 5 hours; and 60mℓ of water was added thereto. The reaction mixture was washed with chloroform until any color did not remain in the chloroform layer. The aqueous layer was separated, decolorized by the filtration through by using active carbon, and concentrated under a reduced pressure. The residue was suspended in a solvent of ethanol and ehtylether(1:2, v/v) to give 330mg of the title compound in white precipitate(yield 65%).

m.p. : 195° ~ 198°C

MS m/z(rel. int, %): 124($M^+$, 7), 107(100), 95(33), 82(53), 80(90)

$^1$H-NMR($CDCl_3$, $\delta$ ppm): 2.1~2.8(4H, m), 3.95(2H, s), 3.96 (2H, s), 4.29(1H, m)

Example 10: Synthesis of 6-methylamino-3-(paratoluenesulfonyl)-3-azabicyclo[3,3,0]-oct-1(5)ene

To a solution of 100mg of 6-amino(N-t-butoxycarbonyl)-3-(paratoluenesulfonyl)-3-azabicyclo[3,3,0]oct-1-(5)ene dissolved in 3mℓ of tetrahydrofuran was added 23mg of lithium aluminium hydride. The resultant mixture was refluxed for 20 minutes; and 10mℓ of methanol and 0.1mℓ of water were added thereto. The reaction mixture was extracted with 50% solution of methanol/dichloromethane(1:1, v/v), concentrated under a reduced pressure and purified by fractionating on the silica gel column chromatography to obtain 70mg of the title compound in white precipitate(yield 83%).

MS m/z(rel, int, %): 292($M^+$)

$^1$H-NMR($CDCl_3$, $\delta$ ppm): 1.89(1H, m), 2.13(1H, m), 2.23(1H, br.s), 2.24(3H, s), 2.46(1H, m), 2.52 (3H, s), 3.66(1H, br.m), 3.84~4.16 (4H, m), 7.27(2H, d, J=8Hz), 7.63(2H, d, J=8Hz)

Example 11: Synthesis of 6-methylamino-3-azabicyclo[3,3,0]oct-1-(5) ene dihydrobromide

A solution of 70mg of 6-methylamino-3-(paratoluene-sulfonyl)-3-azabicyclo[3,3,0]oct-1(5)ene suspended in 0.2mℓ of hydrogen bromide was refluxed for 5 hours; and 10mℓ of water was added thereto. The reaction mixture was washed with chloroform until any color did not remain in the chloroform layer. The aqueous layer was separated, decolorized by being filtered through active carbon, and concentrated under a reduced pressure. The residue was suspended in a solvent of ethanol and ethylether(1:2, v/v) to obtain 40mg of the title compound in white precipitate(yield 55%).

MS m/z(rel. int, %): 138($M^+$, 7), 107(100), 94(18), 80(55), 68(12)

$^1$H-NMR($CDCl_3$, $\delta$ ppm): 2.2~2.8(4H, m), 2.51(3H, s), 3.96 (4H, m), 4.22(1H, br.m)

Example 12: Synthesis of 6-(N-t-butoxycarbonyl-N-ethyl)amino-3-(paratoluenesulfonyl)-3-azabicyclo[3,3,0]-oct-1(5)ene

To a solution of 1.89g of 6-amino(N-t-butoxycarbonyl)-3-(paratoluenesulfonyl)-3-azabicyclo[3,3,0]oct-1-(5)ene dissolved in 20mℓ of dimethylformamide in nitrogen environment was added 280mg of sodium hydride. The resultant mixture was stirred for 2 hours at room temperature; and 850mg of ethyliodide was

added thereto. The reaction mixture was stirred for 5 hours, concentrated under a reduced pressure and purified by fractionating on the silica gel column to obtain 1.73g of the title compound(yield 85%).

$^1$H-NMR($CDCl_3$, δ ppm): 1.45(3H, t, J=7Hz), 1.89~2.05 (2H, m), 2.21(1H, m), 2,46(3H, s), 270(1H, m), 4.01(4H, br.s), 4.20(2H, q, J=7Hz), 4.47(1H, m), 7.30(2H, d, J=8Hz), 7.74-(2H, d, J=8Hz)

Example 13: Synthesis of 6-ethylamino-3-azabicyclo[3,3,0]oct-1(5)-enedihydrobromide

406mg of 6-(N-t-butoxycarbonyl-N-ethyl)amino-3-(paratoluenesulfonyl)-3-azabicyclo[3,3,0]oct-1(5)ene and 100mg of phonol were suspended in 10mℓ of 30% hydrogen bromide solution. The resultant mixture was refluxed for 5 hours, cooled and washed 5 times with 20mℓ of chloroform. The aqueous solution was decolorized by using active carbon, filterted and concentrated. The residue was solidified in a mixture solution of ethanol and ethylether(1:2 v/v) to obtain 200mg of the title compound in white precipitate (yield 63%).

m.p.: 185° ~ 188°C

$^1$H-NMR($CDCl_3$, δ ppm): 1.40(3H, t, J=7Hz), 2.50~2.81 (4H, m), 3.94(4H, br.s), 4.18(2H, q, J=7Hz), 4.24(1H, m)

Example 14: Synthesis of 6-(N-ethyl-N-methyl)amino-3-(paratoluenesulfonyl)-3-azabicyclo[3,3,0]oct-1(5)ene

To a solution of 400mg of 6-(N-t-butoxycarbonyl-N-ethyl) amino-3-(paratoluenesulfonyl)-3-azabicyclo-[3,3,0]oct-1(5)ene dissolved in 10mℓ of tetrahydrofuran was added 100mg of lithium aluminium hydride. The resultant mixture was refluxed for 25 minutes; and 10mℓ of methanol and 0.5mℓ of water were added thereto.

After the reaction mixture was filtered, the filtrate was concentrated under a reduced pressure and purified by fractionating on the silica gel column to obtain 250mg of the title compound (yield 75%)

$^1$H-NMR($CDCl_3$, δ ppm): 1.42(3H, t, J=7Hz), 1.90(1H, m), 2.17~2.20(2H, m), 2.23(3H, s), 2.43 (1H, m), 2.53(3H, s), 3.71(1H, m), 3.95(4H, br.s), 4.24(2H, q, J=7Hz), 7.26(2H, d, J=8Hz), 7.64(2H, d, J=8Hz)

Example 15: Synthesis of 6-(N-ethyl-N-methyl)amino-3-azabicyclo [3,3,0]oct-1(5)ene dihydrobromide

160mg of 6-(N-ethyl-N-methyl)amino-3-(parotoluenesulfonyl)-3-azabicyclo[3,3,0]oct-1(5)ene was suspended in 5mℓ of 30% hydrobromide solution; and the resultant mixture was refluxed for 5 hours followed by addition of 10mℓ of water. The reaction mixture was washed with chloroform; and the aqueous layer was separated, decolorized by being filtered through active carbon and concentrated under a reduced pressure. The residue was suspended in 20mℓ of a mixture of ethanol and ethylether(1:2, v/v); and the precipitates produced therefrom subsequently filtered and dried to obtain 123mg of the title compound(yield 75%).

m.p.: 165° ~ 168°C

$^1$H-NMR($D_2O$, δ ppm): 1.42(3H, t, J=7Hz), 2.25(3H, s), 2.51~2.80(4H, m), 3.90(4H, br.s), 4.15(2H, q, J=7Hz), 4.21(1H, m)

Example 16: Synthesis of 3-bromo-1-cyclohexene-1,2-dicarboxylic acid dimethylester

To a solution of 18g of 1-cyclohexene-1,2-dicarboxylic acid dimethylester dissolved in 180mℓ of carbon tetrachloride were added 16.1g of N-bromosuccinimide and 1.49g of α,α-azobis(isobutyronitrile); and the resultant mixture was refluxed for 5 hours. The reaction mixture was filtered, concentrated under a reduced pressure and fractionated on the silica gel column chromatography to obtain 17.6g of the title compound-(yield 70%).

MS m/z(rel. int, %): 277($M^+$)

$^1$H-NMR($CDCl_3$, δ ppm): 5.23(1H, m), 3.81(6H, s), 3.1(1H, m), 2.7~2.4(3H, m), 1.51(2H, m)

Example 17: Synthesis of 3-azido-1-cyclohexene-1,2-dicarboxylic acid dimethylester

To a solution of 17g of 3-bromo-1-cyclohexene-1,2-dicarboxylic acid dimethylester dissolved in 180mℓ of carbon tetrachloride was added a solution of 14g of sodium azide dissolved in 55mℓ of water; and the resultant mixture was stirred for 16 hours at room temperature. The reaction mixture was refluxed for 1 hour and cooled; and the organic layer was separated, and the remaining aqueous layer was extracted twice with

carbon tetrachloride. All the organic layers were combined, dried over anhydrous magnesium sulfate, and concentrated under a reduced pressure to obtain 13.4g of the title compound in yellow oil(yield 91%).

MS m/z(rel. int, %): 240($M^+$)

$^1$H-NMR($CDCl_3$, δ ppm): 4.79($^1$H, m), 3.79(6H, s), 2.9(1H, m), 2.7(1H, m), 2.41(1H, m), 2.1 (1H, m), 1.45(2H, m)

Example 18: Synthesis of 3-amino-1-cyclohexene-1,2-dicarboxylic acid dimethyl ester

To a solution 13.4g of 3-azido-1-cyclohexene-1,2-dicarboxylic acid dimethylester dissolved in 135mℓ of tetrahydrofuran was added 14.5g of triphenylphosphine. The resultant mixture was stirred for 16 hours under nitrogen gas; and 1.55mℓ of water was added thereto. The reaction mixture was further stirred for 5 hours at a room temperature and concentrated under a reduced pressure; and the residue was dissolved in 240mℓ of dichloromethane. The solution was extracted with 350mℓ of 2N hydrochloric acid solution; and the aqueous layer was separated therefrom and neutralized with 15% NaOH solution. The neutralized solution was extracted with 300mℓ of dichloromethane, dried over anhydrous magnesium sulfate and concentrated under a reduced condition to obtain 5.7g of the title compound in pale yellow oil(yield 48%).

MS m/z(rel. int, %): 213($M^+$)

$^1$H-NMR($CDCl_3$, δ ppm): 4.21(1H, m), 3.73(3H, s), 3.72(3H, s), 3.56(1H, m), 2.75(1H, m), 2.6 (1H, m), 2.41(1H, m), 1.49(2H, m)

Example 19: Synthesis of 3-(N-t-butoxycarbonyl)amino-1-cyclohexene-1,2-dicarboxylic acid dimethylester

To a solution of 5.7g of 3-amino-1-cyclohexene-1,2-dicarboxylic acid dimethylester dissolved in 80mℓ of methanol was added 6.4g of di-t-butylcarbonate. The resultant mixture was stirred for 16 hours at a room temperature, concentrated under a reduced pressure and fractionated on the silica gel column chromatography to obtain 7.95g of the title compound in white precipitate (yield 95%).

MS m/z(rel. int, %): 314($M^+$)

$^1$H-NMR($CDCl_3$, δ ppm): 5.09(1H, br.s), 4.71(1H, br.s), 3.74(3H, s), 3.73(3H, s), 2.75(1H, m), 2.61(1H, m), 2.43(1H, m), 1.48(2H, m), 1.43(9H, s)

Example 20: Synthesis of 3-(N-t-butoxycarbonyl)amino-1,2-bis (hydroxymethyl)-1-cyclohexene

14.4g of diisobutylaluminium hydride was dissolved in 90mℓ of anhydrous tetrahydrofuran; and the solution was cooled to -78°C. Thereafter, a solution of 7.9g of 3-amino(N-t-butoxycarbonyl)amino-1-cyclohexene-1,2-dicarboxylic acid dimethylester dissolved in 80mℓ of tetrahydrofuran was added dropwise to the cooled solution for 1 hour, which was stirred for 16 hours at a room temperautre. 8mℓ of methanol and 2.7mℓ of water were added carefully to the reaction mixture, which was extracted with 200mℓ of 50% solution of methanol/dichloromethane(1:1, v/v) and concentrated under a reduced pressure to obtain 2.78g of the title compound in pale yellow oil (yield 43%).

MS m/z(rel. int, %): 257($M^+$)

$^1$H-NMR($CDCl_3$, δ ppm): 4.69(1H, br.s), 4.31~4.05(5H, m), 2.55~2.25(3H, m), 4.31~4.05(5H, m), 2.55~2.25(3H, m), 1.45(2H, m), 1.41 (9H, s)

Example 21: Synthesis of 3-(N-t-butoxycarbonyl)amino-1,2-bis (bromomethyl)-1-cyclohexene

To a solution of 2.7g of 3-amino(N-t-butoxycarbonyl)amino-1,2-bis(hydroxymethyl)-1-cyclohexene and 8.35g of carbon tetrabromide dissolved in 25mℓ of dichloromethane was added dropwise a solution of 8.1g of triphenylphosphine dissolved in 20mℓ of dichloromethane, in a cold water bath. The resutant solution was stirred for 30 minutes in a cold water bath and concentrated under a reduced pressure; the residue was dissolved in ethylether and filtered to remove undissolved solids; and the filtrate was concentrated under a reduced pressure and fractionated on the silica gel column chromatography to obtain 1.65g of the title compound in white precipitate (yield 41%).

MS m/z(rel. int, %): 383($M^+$)

$^1$H-NMR($CDCl_3$, δ ppm): 4.90(1H, br.s), 4.62(1H, br.s), 4.01(4H, m), 2.7~2.35(3H, m), 1.75~ 1.45(3H, m), 1.43(9H, s)

Example 22: Synthesis of 6-(N-t-butoxycarbonyl)amino-3-(paratoluenesulfonyl)-3-azabicyclo[4,3,0]non-1(5)-ene

To a solution of 1.6g of 3-amino(N-t-butoxycarbonyl)amino-1,2-bis(bromomethyl)-1-cyclohexene and 785mg of paratoluenesulfonamide dissolved in 15mℓ of dimethylformamide was added 416mg of sodium hydride(55~60%) in a cold water bath; and the resultant mixture was stirred for 5 hours. After 60mℓ of water is added, the reaction mixture was extracted with dichloromethane, concentrated in a reduced pressure and fractionated on the silica gel column chromatography to obtain 767mg of the title compound in white precipitate(yield 47%).

| | |
|---|---|
| MS m/z(rel. int, %): | 392($M^+$) |
| $^1$H-NMR($CDCl_3$, δ ppm): | 4.51(1H, br.s), 4.85(1H, br.s), 4.1~3.85(4H, m), 2.61(1H, m), 2.21 (1H, m), 2.11(1H, m), 1.73(1H, m), 1.45(2H, m), 1.39(9H, s) |

Example 23: Synthesis of 6-amino-3-azabicyclo[4,3,0]non-1(5)ene dihydrobromide

To a solution of 760mg of 6-amino(N-t-butoxycarbonyl)amino-3-(paratoluenesulfonyl)-3-azabicyclo-[4,3,0]non-1(5)ene suspended in 2.5mℓ of 30% hydrobormide solution was added 410mg of phenol; and the resultant mixture was refluxed for 5 hours. After 40mℓ of water was added, the reaction mixture was washed with chloroform until any color did not remain in the chloroform layer; and the aqueous layer was filtered through active carbon, concentrated under a reduced pressure and precipitated in 30mℓ of ethanol/ethylether (1:2, v/v) solution to obtain 408mg of the title compound in white precipitate(yield 70%).

| | |
|---|---|
| MS m/z(rel. int, %): | 137($M^+$) |
| $^1$H-NMR($CDCl_3$, δ ppm): | 4.29(1H, br.s), 3.95(4H, s), 2.79 (1H, m), 2.45(1H, m), 2.35(1H, m), 2.22(1H, m), 1.48(2H, m) |

Preparation-

Example 1: Preparation of 7-[6-amino-3-azabicyclo[3,3,0]oct-1(5) ene-3-yl]-1-cyclopropyl-6,8-difluoro-1,4-dihydro-4-oxoquinoline-3-carboxylic acid

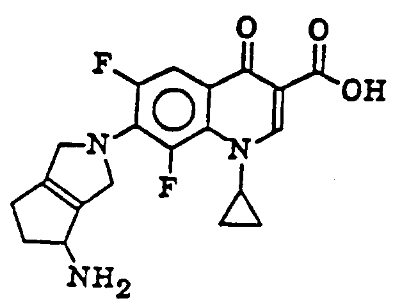

To 10mℓ of acetonitrile was added 330mg of 6-amino-3-azabicyclo[3,3,0]oct-1(5)ene dihydrobromide and 320mg of 1-cyclopropyl-6,7,8-trifluoro-1,4-dihydro-4-oxoquinoline-3-carboxylic acid; and the resulting solution was refluxed for 5 hours after an addition of 616μℓ of 1,8-diazabicyclo[5,4,0]undec-7-ene. The reaction mixture was cooled and filtered; and the residue was washed with acetonitrile to obtain 340mg of the desired compound in light yellow precipitate(yield: 85%).

| | |
|---|---|
| m.p.: | 213~215 °C |
| MS m/z (rel. int, %) | : 387($M^+$),370(100),343(27),326(73),299(25) |
| $^1$H-NMR ($CDCl_3$ + $CD_3COOD$, δ ppm) ; | 8.75 (1H, s), 7.76(1H, dd, J = 14.7, 1.5 Hz), 4.63(4H,br,s)-,4.39 (1H, m),4.1 (1H, m), 4.39 (1H, m), 4.1 (1H, m), 2.90 (1H, m), 2.61 (1H, m), 2.52 (1H, m), 2.35 (1H, m), 1.26(4H, m) |

Preparation Example 2: Preparation of 7-{6-amino-3-azabicyclo[4,3,0] non-1(5)ene-3-yl}-1-cyclopropyl-6,8-difluoro-1,4-dihydro-4-oxoquinoline-3-carboxylic acid (Compound B)

To a mixture of 400mg of 6-amino-3-azabicyclo[4,3,0]non-1(5)ene dihydrobromide and 365mg of 1-cyclopropyl-6,7,8-trifluoro-1,4-dihydro-4-oxoquinoline-3-carboxylic acid suspended in 10mℓ of acetonitrile was added 735μℓ of 1,8-diazabicyclo[5,4,0]undec-7-ene; and the resultant mixture was refluxed for 5 hours. The reaction mixture was cooled and filtered; and the residue was washed with acetonitrile to obtain 387mg of the title compound in pale yellow precipitate(yield 75%).

MS m/z(rel. int, %): 401($M^+$)

$^1$H-NMR($CDCl_3$ + $CD_3COOD$, δ ppm): 8.76(1H, s), 7.73(1H, d, J=13Hz), 4.68(1H, br.s), 4.64(4H, br.s), 4.42(1H, br.s), 4.10(4H, br.s), 2.91 (1H, m), 2.65(1H, m), 2.55 (1H, m), 2.35(1H, m), 1.47 (2H, m), 1.25(4H, m)

In vitro anti-bacterial activity test

Quinolone compounds may be prepared by using a compound of formula(I) as shown in Preparation Examples. In order to demonstrate the effectiveness of the quinolone compound prepared by using a compound of the present invention, the minimal inhibitory concentration(MIC)s of compounds A and B was determined and compared with that of ciprofloxacin. These MIC values were taken by employing two-fold dilution method; that is, two-fold serial dilutions of each of the test compounds were made and dispersed in a Mueller-Hinton agar medium; 2μℓ of the standard strain which had the $10^7$ CFU(colon forming unit) per mℓ was inoculated on the medium; and these were incubated at 37°C for 20 hours. The result of the MIC tests are shown in Table 1.

Table 1

| In vitro anti-bacterial activity test(MIC, μg/mℓ) | | | |
|---|---|---|---|
| Strain | Compound A | Compound B | Ciprofloxacin |
| Streptococcus pyogenes 308 | 0.195 | 0.195 | 3.125 |
| Streptococcus pyogenes 77 | 0.098 | 0.098 | 1.563 |
| Streptococcus faecium MD 8b | 0.098 | 0.098 | 0.781 |
| Staphylococcus aureus SG 511 | 0.025 | 0.049 | 0.195 |
| Staphylococcus aureus 285 | 0.049 | 0.049 | 0.781 |
| Staphylococcus aureus 503 | 0.049 | 0.049 | 0.781 |
| Escherichia coli 055 | 0.013 | 0.013 | 0.013 |
| Escherichia coli DC 0 | 0.391 | 0.391 | 0.195 |
| Escherichia coli DC 2 | 0.098 | 0.098 | 0.098 |
| Escherichia coli TEM | 0.049 | 0.098 | 0.025 |
| Escherichia coli 1507E | 0.049 | 0.049 | 0.025 |
| Pseudomonas aeruginosa 9027 | 0.781 | 1.563 | 0.391 |
| Pseudomonas aeruginosa 1592E | 0.781 | 0.781 | 0.195 |
| Pseudomonas aeruginosa 1771 | 0.781 | 0.781 | 0.391 |
| Pseudomonas aeruginosa 1771M | 0.391 | 0.781 | 0.098 |
| Salmonella typhimurium | 0.049 | 0.049 | 0.025 |
| Klebsiella aerogenes 1082E | 0.049 | 0.049 | 0.013 |
| Klebsiella aerogenes 1522E | 0.098 | 0.049 | 0.025 |
| Enterobacter cloacae P 99 | 0.025 | 0.013 | 0.025 |
| Enterobacter cloacae 132E | 0.025 | 0.025 | 0.025 |

Preparation-

Example 2b;Preparation of 1-cyclopropyl-6,8-difluoro-7-[6-methylamino-3-azabicyclo[3,3,0]oct-1(5)ene-3-yl]-1,4-dihydro-4-oxoquinoline-3-carboxylic acid

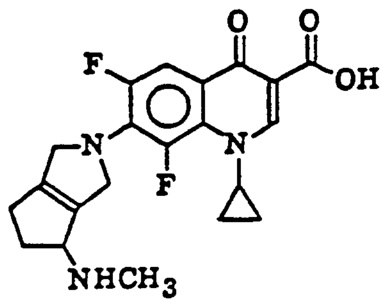

To 3mℓ of acetonitrile was added 40mg of 6-methylamino-3-azabicyclo[3,3,0]oct-1(5)ene dihydrobromide and 37.5mg of 1-cyclopropyl-6,7,8-trifluoro-1,4-dihydro-4-oxoquinoline-3-carboxylic acid; and the resulting solution was refluxed for 5 hours after an addition of 72μℓ of 1,8-diazabicyclo[5,4,0]undec-7-ene. The reaction mixture was cooled and filtered; and the residue was washed with acetonitrile to obtain 34mg of the desired compound in white precipitate(yield: 65%).

| | |
|---|---|
| m.p.: | 194~196 °C |
| MS m/z (rel. int, %): | 401($M^+$), 370(89), 326(100), 299(15), 285(10), 258(10), 237-(10), 220(20) |
| $^1$H-NMR ($CDCl_3$ + $CD_3COOD$, δ ppm) ; | 8.74 (1H, s), 7.73 (1H, d, J = 14 Hz), 4.67 (4H, br, m), 4.39 (1H, br, s), 4.09 (1H, m), 2.85(1H, m), 2.71 93H, s), 2.61(1H, m), 2.50(1H, m), 2.42(1H, m), 1.21(4H, m) |

Preparation-

Example 3: Preparation of 7-[6-amino-3-azabicyclo[3,3,0]oct-1(5) ene-3-yl]-1-cyclopropyl-8-chloro-6-fluoro-1,4-dihydro-4-oxoquinoline-3-carboxylic acid

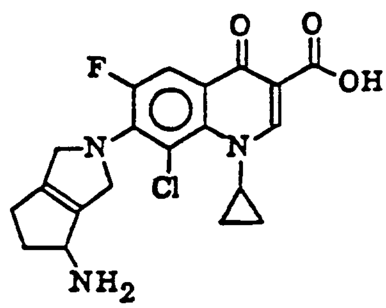

To 25mℓ of acetonitrile was added 800mg of 6-amino-3-azabicyclo[3,3,0]oct-1(5)ene dihydrobromide and 790mg of 1-cyclopropyl-6,7-difluoro-8-chloro-1,4-dihydro-4-oxoquinoline-3-carboxylic acid; and the resulting solution was refluxed for 5 hours after an addition of 1.38mℓ of 1,8-diazabicyclo[5,4,0]undec-7-ene. The reaction mixture was cooled and filtered; and the residue was washed with acetonitrile to obtain 400mg of the desired compound in white color(yield: 38%).

| | |
|---|---|
| m.p.: | 193~194 °C |
| MS m/z (rel. int, %): | 403($M^+$, 10), 386(72), 359(27), 342(100), 307(62), 282(65), 252(51), 216(47), 201(32), 172(27), 108(35) |
| $^1$H-NMR ($CDCl_3$ + $CD_3COOD$, δ ppm) ; | 8.97 (1H, s), 8.03 (1H, d, J = 12 Hz), 4.46~4.34 (6H, m), 2.80 (1H, br, s), 2.61 (1H, br, s), 2.46 (2H, br, s), 1.32 (2H, m), 0.98 (2H, m) |

Preparation-

Example 4: Preparation of 7-[6-methylamino-3-azabicyclo[3,3,0]oct-1(5)ene-3-yl]-8-chloro-1-cyclopropyl-6-fluoro-1,4-dihydro-4-oxoquinoline-3-carboxylic acid

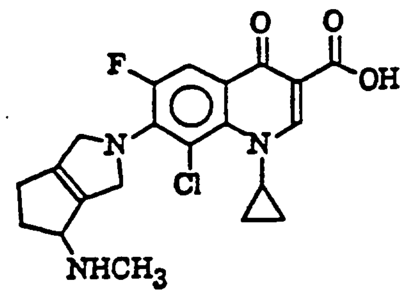

To 2.5mℓ of acetonitrile was added 100mg of 6-methylamino-3-azabicyclo[3,3,0]oct-1(5)ene dihydrobromide and 95mg of 1-cyclopropyl-6,7-difluoro-1,4-dihydro-4-oxoquinoline-3-carboxylic acid; and the resulting solution was refluxed for 4 hours after an addition of 164μℓ of 1,8-diazabicyclo[5,4,0]undec-7-ene. The reaction mixture was cooled and filtered; and the residue was washed with acetonitrile to obtain 80mg of the desired compound in white color(yield: 60%).

| | |
|---|---|
| m.p.: | 207~208 °C |
| MS m/z (rel. int, %) : | 417 ($M^+$, 6), 386(90), 342(100), 308(46), 288(29), 263(22), 216(27), 122(50), 109(68) |
| $^1$H-NMR ($CDCl_3$ + $CD_3COOD$, δ ppm) ; | 8.95 (1H, s), 8.04 (1H, d, J = 14.4 Hz), 4.46~4.33 (6H, br, m), 2.7 (3H, s), 2.82~2.49 (4H, m), 1.32(2H, m), 0.98 (2H, m) |

Preparation-

Example 5: Preparation of 7-[6-amino-3-azabicyclo[3,3,0]oct-1(5) ene-3-yl]-8-bromo-1-cyclopropyl-6-fluoro-1,4-dihydro-4-oxoquinoline-3-carboxylic acid

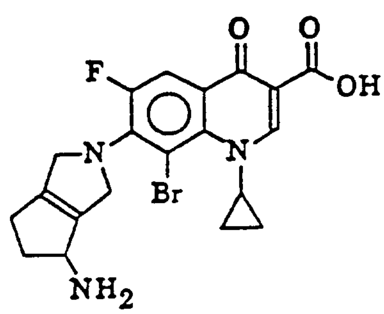

320mg of 6-amino-3-azabicyclo[3,3,0]oct-1(5)ene dihydrobromide and 344mg of 8-bromo-1-cyclopropyl-6,7-difluoro-1,4-dihydro-4-oxoquinoline-3-carboxylic acid were subjected to the same process as described in Example 1 to obtain 271mg of the desired compound (yield: 70%).

| | |
|---|---|
| m.p.: | 209~213 °C |
| $^1$H-NMR ($CDCl_3$ + $CD_3COOD$, δ ppm) ; | 8.15 (1H, s), 7.49 (1H, d, J=12.0 Hz), 4.48 (4H, br. s), 4.35 (1H, m), 4.01 (1H, m), 2.92 (1H, m), 2.63 (1H, m), 2.50 (1H, m), 2.34 (1H, m), 1.20~1.05 (4H, m) |

Preparation-

Example 6: Preparation of 7-[6-amino-3-azabicyclo[3,3,0]oct-1(5) ene-3-yl]-1-cyclopropyl-6-fluoro-8-methoxy-1,4-dihydro-4-oxoquinoline-3-carboxylic acid

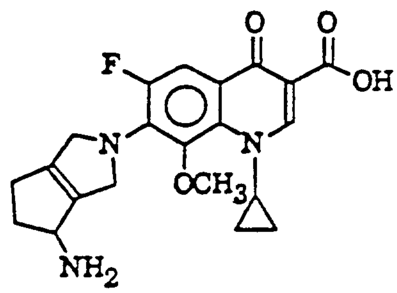

320mg of 6-amino-3-azabicyclo[3,3,0]oct-1(5)ene dihydrobromide and 295mg of 1-cyclopropyl-6,7-difluoro-8-methoxy-1,4-dihydro-4-oxoquinoline-3-carboxylic acid were subjected to the same process as described in Example 1 to obtain 232mg of the desired compound (yield: 58%).

| | |
|---|---|
| m.p.: | 195~198°C |
| $^1$H-NMR ($CDCl_3$ + $CD_3COOD$, δ ppm) ; | 8.84 (1H, s), 7.69 (1H, d, J = 13.8 Hz), 4.55~4.35 (5H, br, s), 4.05 (1H, m), 3.59 (3H, s), 2.95~2.30 (4H, m), 1.23~1.05 (4H, m) |

Preparation-

Example 7: Preparation of 7-[6-methylamino-3-azabicyclo[3,3,0]oct-1(5)ene-3-yl]-1-cyclopropyl-6-fluoro-1,4-dihydro-4-oxo-1,8-naphthyridine-3-carboxlic acid

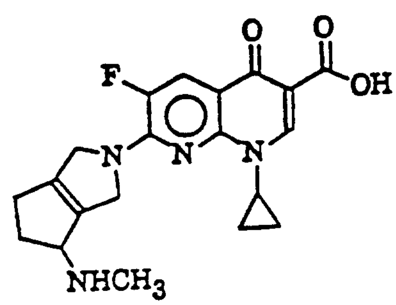

To 2mℓ of acetonitrile was added 70mg of 6-methylamino-3-azabicyclo[3,3,0]oct-1(5)ene dihydrobromide and 62mg of 1-cyclopropyl-7-chloro-6-fluoro-4-oxo-1,8-naphthyridine-3-carboxylic acid; and the resulting solution was refluxed for 4 hours after an addition of 62mg of 1,8-diazabicyclo[5,4,0]undec-7-ene. The reaction mixture was cooled, concentrated under a reduced pressure and fractionated on silica gel column chromatography(eluent:chloroform/methanol/water = 15/3/1) to obtain 60mg of the desired compound in yellow color (yield: 71%).

| | |
|---|---|
| m.p.: | 185~ 190°C |
| MS m/z (rel. int, %): | 384 ($M^+$, 8), 353 (17), 342 (100), 311 (62), 296 (55), 284 (15), 218 (15), 163 (10), 122 (15), 109 (15) |
| $^1$H-NMR ($CDCl_3$ + $CD_3COOD$, δ ppm) ; | 8.69 (1H, s), 7.98 (1H, br, s), 4.63~4.45 (5H, br, m) 3.64 (1H, br, s), 2.68 (3H, s), 2.8~2.45 (4H, m), 1.28 (2H, br, s), 1.07 (2H, br, s) |

Preparation-

Example 8: Preparation of 7-[6-amino-3-azabicyclo[3,3,0]oct-1(5) ene-3-yl]-6,8-difluoro-1-ethyl-1,4-dihydro-4-oxoquinoline-3-carboxylic acid

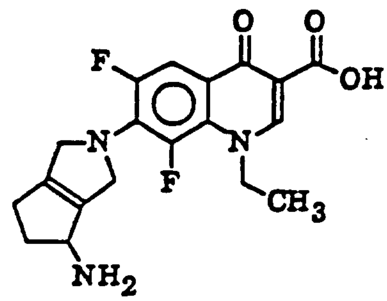

320mg of 6-amino-3-azabicyclo[3,3,0]oct-1(5)ene dihydrobromide and 343mg of 1-ethyl-6,7,8-trifluoro-1,4-dihydro-4-oxoquinoline-3-carboxylic acid were subjected to the same process as described in Example 1 to obtain 291mg of the desired compound (yield: 65%).

| | |
|---|---|
| m.p.: | 245~ 249 ° C (decomposition) |
| $^1$H-NMR ($CDCl_3$ + $CD_3COOD$, δ ppm) ; | 8.78 (1H,s), 7.86 (1H, d, J = 14.2 Hz), 4.60 (4H, br.s), 4.31 (1H, m), 4.23 (2H, q, J = 7 Hz), 2.94 (1H, m), 2.64 (1H, m), 2.51 (1H, m), 2.35 (1H, m), 1.45 (3H, t, J = 7 Hz) |

Preparation-

Example 9: Preparation of 7-[6-amino-3-azabicyclo[4,3,0]non-1(5) ene-3-yl]-1-cyclopropyl-6,8-difluoro-1,4-dihydro-4-oxoquinoline-3-carboxylic acid

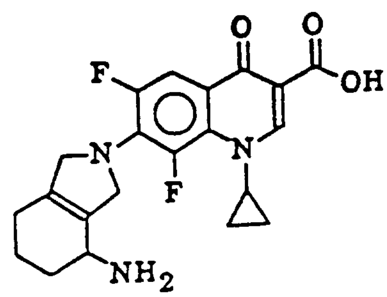

To 10mℓ of acetonitrile was added 400mg of 6-amino-3-azabicyclo[4,3,0]non-1(5)ene dihydrobromide and 365mg of 1-cyclopropyl-6,7,8-trifluoro-4-oxoquinoline-3-carboxylic acid; and the resulting solution was refluxed for 5 hours after an addition of 735μℓ of 1,8-diazabicyclo[5,4,0]undec-7-ene. The reaction mixture was cooled and filtered; and the residue was washed with acetonitrile to obtain 387mg of the desired compound in light yellow precipitate (yield: 75%).

| | |
|---|---|
| m.p,: | 200~ 204 ° C |
| $^1$H-NMR ($CDCl_3$ + $CD_3COOD$, δ ppm) ; | 8.76 (1H,s), 7.73 (1H, d, J = 13Hz), 4.68 (1H, br, s), 4.64 (4H, br, s), 4.42 (1H, br, s), 4.10 (1H, br, s), 2.91 (1H, m), 2.65 (1H, m), 2.55 (1H, m), 2.35 (1H, m), 1.47 (2H, m), 1.25 (4H, m) |

Preparation-

Example 10: Preparation of 7-[6-amino-3-azabicyclo[3,3,0]oct-1(5) ene-3-yl]-5-amino-1-cyclopropyl-6,8-difluoro-1,4-dihydro-4-oxoquinoline-3-carboxylic acid

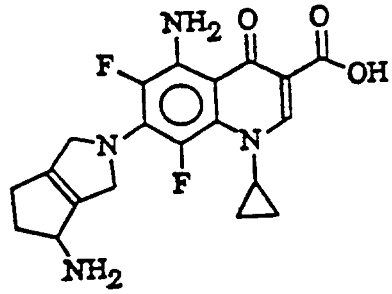

To 2mℓ of acetonitrile was added 50mg of 6-amino-3-azabicyclo[3,3,0]oct-1(5)ene dihydrobromide and 50mg of 5-amino-1-cycloporpyl-6,7,8-trifluoro-1,4-dihydro-4-oxoquinoline-3-carboxylic acid; and the resulting solution was refluxed for 5 hours after an addition of 86$\mu$l of 1,8-diazabicyclo[5,4,0]undec-7-ene. The reaction mixture was cooled and filtered; and the residue was washed with acetonitrile to obtain 35mg of the desired compound in light yellow color(yield: 50%).

| | |
|---|---|
| m.p.: | 134~ 137°C |
| MS m/z (rel. int, %) : | 407 ($M^+$, 23), 385 (14), 358 (45), 295 (23), 231 (15), 50 (100), 137 (40) |
| $^1$H-NMR ($CDCl_3$ + $CD_3COOD$, $\delta$ ppm) ; | 8.63 (1H, s), 4.55 (5H, br, s), 3.91 (1H, br, s), 2.78~2.43 (4H, br, m), 1.18 (2H, br, s), 1.04 (2H, br, s) |

Preparation-

Example 11: Preparation of 7-[6-methylamino-3-azabicyclo[3,3,0]oct-1(5)ene-3-yl)-5-amino-1-cyclopropyl-6,8-difluoro-1,4-dihydro-4-oxoquinoline-3-carboxylic acid

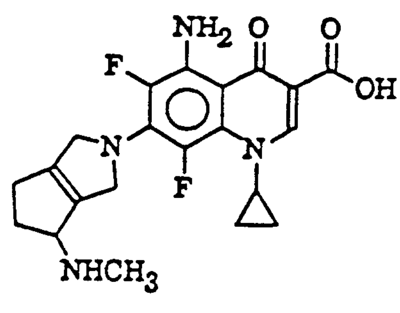

To 2mℓ of acetonitrile was added 70mg of 6-methylamino-3-azabicyclo[3,3,0]oct-1(5)ene dihydrobromide and 66mg of 5-amino-1-cyclopropyl-6,7,8-trifluoro-1,4-dihydro-4-oxoquinoline-3-carboxylic acid; and the resulting solution was refluxed for 4 hours after an addition of 115$\mu$ℓ of 1,8-diazabicyclo-[5,4,0]undec-7-ene. The reaction mixture was cooled and filtered; and the residue was washed with acetonitrile to obtain 73mg of the desired compound in light yellow color(yield: 80%).

| | |
|---|---|
| m.p.: | 222~ 224°C |
| MS m/z (rel. ink %) : | 416 ($M^+$, 95), 385 (100), 372 (37), 320 (38), 300 (45), 231 (87), 216 (25), 122 (56), 109 (57) |
| $^1$H-NMR ($CDCl_3$ + $CD_3COOD$, $\delta$ ppm) ; | 8.63 (1H, s), 4.65~4.41 (5H, m), 3.90 (1H, br, s), 2.68 (3H, s), 2.80~2.44 (4H, m), 1.19 (2H, m), 1.05 (2H, m) |

Preparation-

Example 12: Preparation of 7-[6-amino-3-azabicyclo[3,3,0]oct-1(5) ene-3-yl]-1-(2,4-difluorophenyl)-6,8-difluoro-1,4-dihydro-4-oxoquinoline-3-carboxylic acid

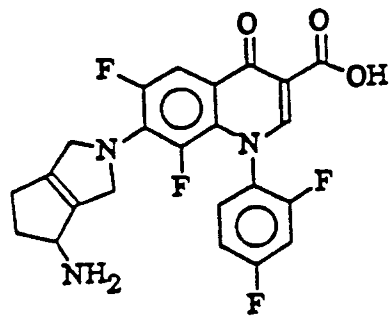

To 1.2mℓ of acetonitrile was added 65mg of 6-amino-3-azabicyclo[3,3,0]oct-1(5)ene dihydrobromide and 70mg of 1-(2,4-difluorophenyl)-6,7,8-trifluoro-1,4-dihydro-4-oxoquinoline-3-carboxylic acid; and the resulting solution was refluxed for 4 hours after an addition of 91μℓ of 1,8-diazabicyclo[5,4,0]undec-7-ene. The reaction mixture was cooled and filtered; and the residue was washed with acetonitrile and ethylether to obtain 45mg of the desired compound(yield 50%).

| | |
|---|---|
| m.p: | 185~ 190 °C (decomposition) |
| MS m/z (rel. int, %) : | 459 ($M^+$, 1), 442 (3), 415 (30), 398 (100), 371 (40), 321 (15), 292 (17), 151 (10), 123 (14) |
| $^1$H-NMR ($CDCl_3$ + $CD_3COOD$, δ ppm) ; | 8.49 (1H, s), 7.91 (1H, d, J = 14 Hz), 8.17 (1H, m), 7.08 (2H, m), 4.50~4.30 (5H, m), 2.71~2.32 (4H, m) |

Preparation-

Example 13: Preparation of 7-[6-methylamino-3-azabicyclo[3,3,0]oct-1(5)ene-3-yl]-1-(2,4-difluorophenyl)-6,8-difluoro-1,4-dihydro-4-oxoquinoline-3-carboxylic acid

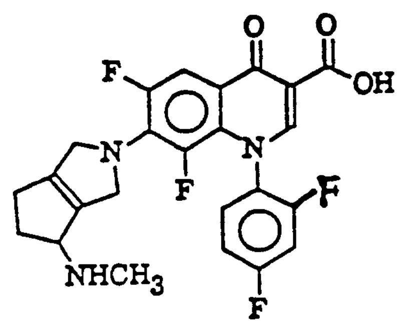

To 1.2mℓ of acetonitrile was added 65mg of 6-methylamino-3-azabicyclo[3,3,0]oct-1(5)ene dihydrobromide and 70mg of 1-(2,4-difluorophenyl)-6,7,8-trifluoro-1,4-dihydro-4-oxoquinoline-3-carboxylic acid; and the resulting solution was refluxed for 4 hours after an addition of 91μℓ of 1,8-diazabicyclo[5,4,0]-undec-7-ene. The reaction mixture was cooled and filtered; and the residue was washed with acetonitrile and ethylether to obtain 60mg of the desired compound(yield: 65%).

| | |
|---|---|
| m.p.: | 228~ 300 °C |
| MS m/z (rel. int, %) : | 473 ($M^+$, 3), 441 (13), 430 (25), 399 (100), 372 (23), 307 (10, 237 (6), 122 (10), 109 (15), 94 (10) |
| $^1$H-NMR ($CDCl_3$ + $CD_3COOD$, δ ppm) | ; 8.48 (1H, s), 7.89 (1H, dd, J = 13, 1.5 Hz), 7.50 (1H, m), 7.08 (2H, m), 4.56~4.31 (5H, m), 2.74~2.39 (4H, m), 2.62 (3H, |

s)

Preparation-

Example 14: Preparation of 7-[6-amino-3-azabicyclo[3,3,0]oct-1(5) ene-3-yl]-1-cyclopropyl-6-fluoro-1,4-dihydro-4-oxo-1,8-naphthyridine-3-carboxylic acid

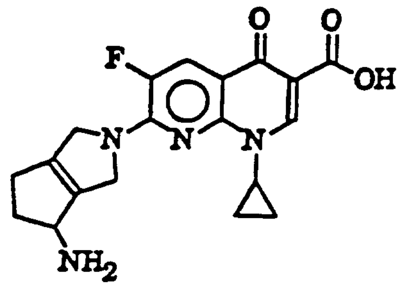

320mg of 6-amino-3-azabicyclo[3,3,0]oct-1(5)ene dihydrobromide and 282mg of 1-cyclopropyl-7-chloro-6-fluoro-4-oxo-1,8-naphthyridine-3-carboxylic acid were subjected to the same process as described in Example 1 to obtain 229mg of the desired compound (yield: 59%).

| | |
|---|---|
| m.p.: | 175~ 179 °C |
| $^1$H-NMR ($CDCl_3$ + $CD_3COOD$, $\delta$ ppm) ; | 8.72 (1H, s), 7.82 (1H, br, s), 4.71~4.52 (5H, br, m), 3.61 (1H, br, s), 2.8~2.39 (4H, m), 1.28 (2H, br, s), 1.05 (2H, br, s) |

Preparation-

Example 15: Preparation of 7-[6-methylamino-3-axabicyclo[3,3,0]oct-1(5)ene-3-yl]-1-cyclopropyl-6-fluoro-1,4-dihydro-4-oxo-1,8-naphthyridine-3-carboxylic acid

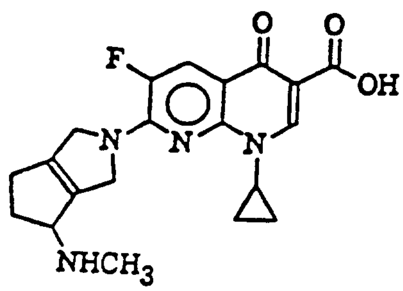

To 2mℓ of acetonitrile was added 70mg of 6-methylamino-3-azabicyclo[3,3,0]oct-1(5)ene dihydrobronide and 62mg of 1-cyclopropyl-7-chloro-6-fluoro-4-oxo-1,8-naphthyridine-3-carboxylic acid; and the resulting solution was refluxed for 4 hours after an addition of 62mg of 1,8-diazabicyclo[5,4,0]undece-7-ene. The reaction mixture was cooled, concentrated under a reduced pressure and fractionated on silica gel column chromatography(eluent: chloroform/methanol/water = 15/3/1) to obtain 60mg of the desired compound in yellow color(yield: 71%).

| | |
|---|---|
| m.p.: | 185~ 190 °C |
| MS m/z (rel. int, %) : | 384($M^+$, 8), 353 (17), 342 (100), 311 (62), 296 (55), 284 (15), 218 (15), 163 (10), 122 (15), 109 (15) |
| $^1$H-NMR ($CDCl_3$ + $CD_3COOD$, $\delta$ ppm) ; | 8.69 (1H, s), 7.98 (1H, br, s), 4.63~4.45 (5H, br, m), 3.64 (1H, br, s), 2.68 (3H, s), 2.8~2.45 (4H, m), 1.28 (2H, br, s), 1.07 (2H, br, s) |

Preparation-

Example 16: Preparation of 9-fluoro-10-[6-amino-3-azabicyclo[3,3,0] oct-1(5)ene-3-yl]-3-(S)-methyl-7-oxo-2,3-dihydro-7H-pyrido[1,2,3-de]-1,4-benzoxazine-6-carboxylic acid

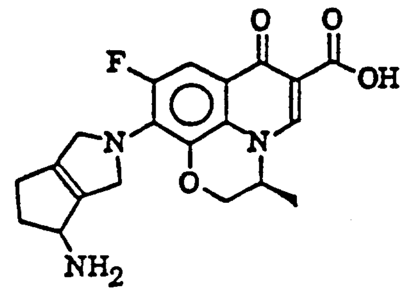

320mg of 6-amino-3-azabicyclo[3,3,0]oct-1(5)ene dihydrobromide and 295mg of 9,10-difluoro-3-(S)-methyl-7-oxo-2,3-dihydro-7H-pyrido[1,2,3-de]-1,4-benzoxazine-6-carboxylic acid were subjected to the same process as described in Example 1 to obtain 196mg of the desired compound(yield: 49%).

| | |
|---|---|
| m.p.: | 183~ 186 ° C |
| MS m/z (ret. int, %) : | 399 ($M^+$, 7), 368 (40), 357 (27), 326 (100), 234 (57), 219 (67), 205 (35), 193 (31), 122 (44), 109 (47) |
| $^1$H-NMR ($CDCl_3$ + $CD_3COOD$, δ ppm) ; | 8.75 (1H, s), 7.65 (1H, d, J = 14.2 Hz), 4.65~4.30 (6H, m), 4.25 (2H, br, s), 2.98~2.35 (4H, m), 1.67 (3H, d, J = 6.5 Hz) |

Preparation-

Example 17: Preparation of 9-fluoro-10-[6-methylamino-3-azabicyclo [3,3,0]oct-1(5)ene-3-yl]-3-(S)-methyl-7-oxo-2,3-dihydro-7H-pyrido[1,2,3-de]-1,4-benzoxazine-6-carboxylic acid

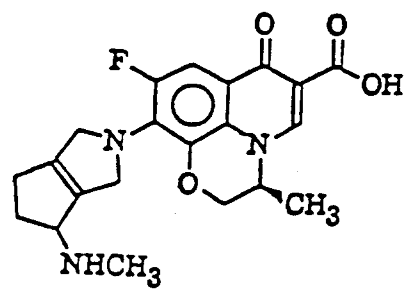

To 1.5mℓ of acetonitrile was added 70mg of 6-methylamino-3-azabicyclo[3,3,0]oct-1(5)ene dihydrobromide and 63mg of 9,10-difluoro-3-(S)-methyl-7-oxo-2,3-dihydro-7H-pyrido[1,2,3-de]-1,4-benzeoxazine-6-carboxylic acid; and the resulting solution was refluxed for 4 hours after an addition of 115μℓ of 1,8-diazabicyclo [5,4,0]undece-7-ene. The reaction mixture was cooled, filterted and washed with acetonitrile to obtain 45mg of the desired compound (yield: 50%).

| | |
|---|---|
| m.p.: | 186~ 188 ° C |
| MS m/z (rel. int, %): | 399 ($M^+$, 7), 368 (40), 357 (27), 326 (100), 234 (57), 219 (67), 205 (35), 193 (31), 122 (44), 109 (47) |
| $^1$H-NMR ($CDCl_3$ + $CD_3COOD$, δ ppm) ; | 8.78 (1H, s), 7.68 (1H, dd, J = 13 Hz, 1.2 Hz), 4.67~4.28 (8H, m), 2.67 (3H, s), 2.80~2.47 (4H, m), 1.61 (3H, d, J = 6.7 Hz) |

Preparation-

Example 18: Preparation of 7-[6-amino-3-azabicyclo[3,3,0]oct-1(5) ene-3-yl]-1-ethyl-6-fluoro-1,4-dihydro-4-oxoquinoline-3-carboxylic acid

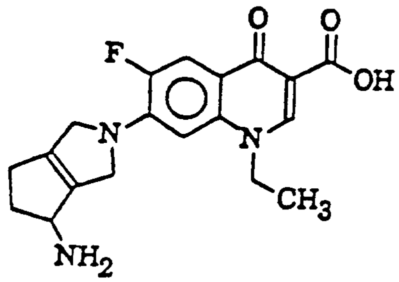

320mg of 6-amino-3-azabicyclo[3,3,0]oct-1(5)ene dihydrobromide and 252mg of 6,7-difluoro-1-ethyl-1,4-dihydro-4-oxoquinoline-3-carboxylic acid were subjected to the same process as described in Example 1 to obtain 260mg of the desired compound(yield: 73%).

| | |
|---|---|
| m. p.: | 212~ 215 °C |
| $^1$H-NMR ($CDCl_3$ + $CD_3COOD$, δ ppm) ; | 8.75 (1H, s), 7.85 (1H, d, J = 12.4 Hz), 7.12 (1H, d, J = 7.3 Hz), 4.45 (4H, br, s), 4.31 (1H, m), 4.09 (2H, q, J = 7 Hz), 2.42 (2H, m), 2.18 (2H, m), 1.42 (3H, t, J = 7 Hz) |

Preparation-

Example 19: Preparation of 7-[6-methylamino-3-azabicyclo[3,3,0] oct-1(5)ene-3-yl]-1-ethyl-6-fluoro-1,4-dihydro-4-oxoquinoline-3-carboxylic acid

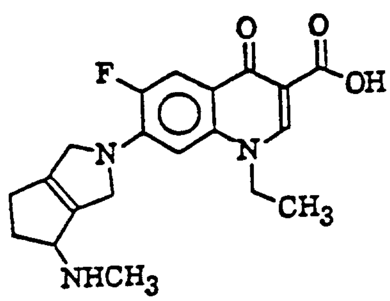

350mg of 6-methylamino-3-azabicyclo[3,3,0]oct-1(5)ene dihydrobromide and 252mg of 6,7-difluoro-1-ethyl-1,4-dihydro-4-oxoquinoline-3-carboxylic acid were subjected to the same process as described in Example 1 to obtain 241mg of the desired compound (yield: 65%).

| | |
|---|---|
| m.p.: | 220~ 224 °C |
| $^1$H-NMR ($CDCl_3$ + $CD_3COOD$, δ ppm) ; | 8.69 (1H, s), 7.76 (1H, d, J = 12.4 Hz), 7.69 (1H, d, J = 7.3 Hz), 4.50~4.39 (5H, br, s), 4.05 (2H, q, J = 7 Hz), 2.91 (1H, m), 2.70 (3H, s), 2.49~2.20 (3H, br, s), 1.41 (3H, t, J = 7 Hz) |

Preparation-

Example 20: Preparation of 7-[6-amino-3-azabicyclo[3,3,0]oct-1(5) ene-3-yl]-1-ethyl-6-fluoro-1,4-dihydro-4-oxoquinoline-3-carboxylic acid

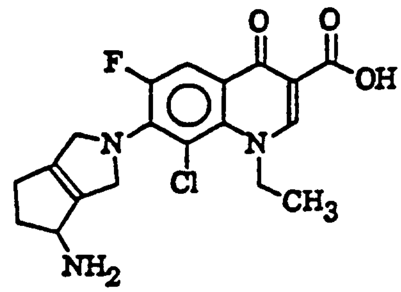

320mg of 6-amino-3-azabicyclo[3,3,0]oct-1(5)ene dihydrobromide and 286mg of 1-ethyl-6,7-difluoro-8-chloro-1,4-dihydro-4-oxoquinoline-3-carboxylic acid were subejcted to the same process as described in Example 1 to obtain 246mg of the desired compound (yield: 63%).

| | |
|---|---|
| m.p.: | 218~ 221 ° C |
| $^1$H-NMR ($CDCl_3$ + $CD_3COOD$, δ ppm) ; | 8.95 (1H, s), 7.98 (1H, d, J = 14.1 Hz), 4.58 (5H, br, s), 4.21 (2H, q, J = 7 Hz), 2.62 (1H, m), 2.51 (2H, m), 2.31 (1H, m), 1.44 (3H, t, J = 7 Hz) |

Preparation-

Example 21: Preparation of 7-[6-methylamino-3-azabicyclo[3,3,0]oct-1(5)ene-3-yl]-1-ethyl-6-fluoro-8-chloro-1,4-dihydro-4-oxoquinoline-3-carboxylic acid

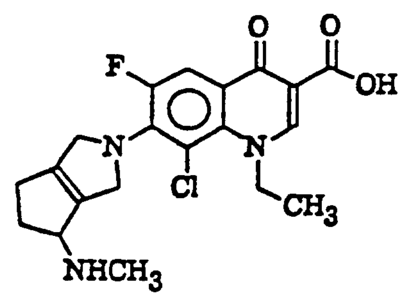

350mg of 6-methylamino-3-azabicyclo[3,3,0]oct-1(5)ene dihydrobromide and 286mg of 1-ethyl-6,7-difluoro-8-chloro-1,4-dihydro-4-oxoquinoline-3-carboxylic acid were subjected to the same process as described in Example 1 to obtain 223mg of the desired compound (yield: 55%).

| | |
|---|---|
| m.p.: | 223~ 227 ° C |
| $^1$H-NMR ($CDCl_3$ + $CD_3COOD$, δ ppm) ; | 8.93 (1H, s), 7.89 (1H, d, J = 14.1 Hz), 4.59~4.39 (5H, br, s), 4.20 (2H, q, J = 7 Hz), 2.69 (3H, s), 2.60 (1H, m), 2.53 (2H, m), 2.30 (1H, m), 1.43 (3H, t, J = 7 Hz) |

Preparation-

Example 22: Preparation of 7-[6-amino-3-azabicyclo[3,3,0]oct-1(5) ene-3-yl]-1-ethyl-5-amino-6,8-difluoro-1,4-dihydro-4-oxoquinoline-3-carboxylic acid

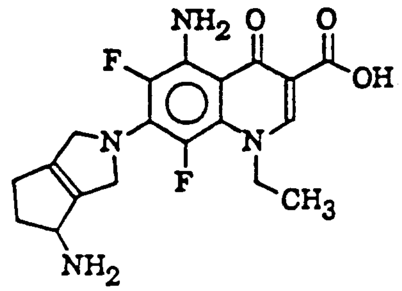

320mg of 6-amino-3-azabicyclo[3,3,0]oct-1(5)ene dihydrobromide and 286mg of 1-ethyl-5-amino-6,7,8-trifluoro-1,4-dihydro-4-oxoquinoline-3-carboxylic acid were subjected to the same process as described in Example 1 to obtain 242mg of the desired compound (yield: 62%).

| | |
|---|---|
| m.p.: | 238~ 243 °C |
| $^1$H-NMR ($CDCl_3$ + $CD_3COOD$, $\delta$ ppm) ; | 8.71 (1H, s), 4.52 (4H, br, s), 4,31 (1H, m), 4.21 (2H, t, J = 7 Hz), 2.92 (1H, m), 2.64 (1H, m), 2.48 (1H, m), 2.33 (1H, m), 1.42 (3H, t, J = 7 Hz) |

Preparation-

Example 23: Preparation of 7-[6-methylamino-3-azabicyclo[3,3,0]oct-1(5)ene-3-yl]-1-ethyl-5-amino-6,8-difluoro-1,4-dihydro-4-oxoquinoline-3-carboxylic acid

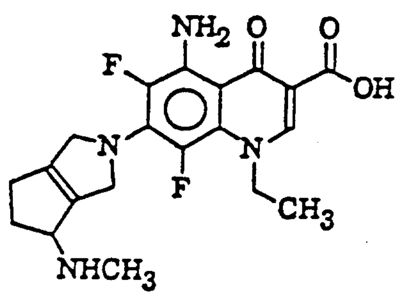

350mg of 6-methylamino-3-azabicyclo[3,3,0]oct-1(5)ene dihydrobromide and 286mg of 1-ethyl-5-amino-6,7,8-trifluoro-1,4-dihydro-4-oxoquinoline-3-carboxylic acid were subjected to the same process as described in Example 1 to obtain 223mg of the desired compound(yield: 55%).

| | |
|---|---|
| m.p.: | 218~ 221 °C |
| $^1$H-NMR ($CDCl_3$ + $CD_3COOD$, $\delta$ ppm) ; | 8.69 (1H, s), 4.50~4.41 (5H, br, s), 4.18 (2H, t, J = 7 Hz), 2.90 (1H, m), 2.70 (3H, s), 2.65 (1H, m), 2.47 (1H, m), 2.31 (1H, m), 1.38 (3H, t, J = 7 Hz) |

Preparation-

Example 24: Preparation of 7-[6-amino-3-azabicyclo[3,3,0]oct-1(5) ene-3-yl]-1-(t-butyl)-6,8-difluoro-1,4-dihydro-4-oxoquinoline-3-carboxylic acid

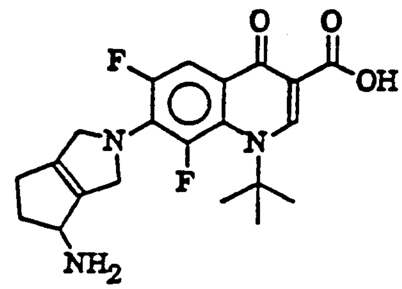

330mg of 6-amino-3-azabicyclo[3,3,0]oct-1(5)ene dihydrobromide and 299mg of 6,7,8-tirfluoro-1-(t-butyl)-1,4-dihydro-4-oxoquinoline-3-carboxylic acid were subjected to the same process as described in Example 1 to obtain 222mg of the desired compound (yield: 55%).

| | |
|---|---|
| m.p.: | 189~ 192 °C |
| $^1$H-NMR ($CDCl_3$ + $CD_3COOD$, δ ppm) ; | 8.58 (1H, s), 8.12 (1H, d, J = 14.2 Hz), 4.51~4.35 (5H, m), 2.91 (1H, m), 2.60~2.45 (2H, m), 2.31 (1H, m), 1.83 (9H, s) |

Preparation-

Example 25: Preparation of 7-[6-methylamino-3-azabicyclo[3,3,0]oct-1(5)ene-3-yl]-1-(t-butyl)-6,8-difluoro-1,4-dihydro-4-oxoquinoline-3-carboxylic acid

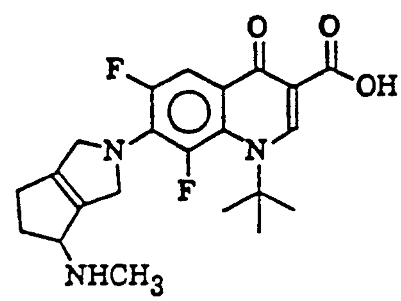

355mg of 6-methylamino-3-azabicyclo[3,3,0]oct-1(5)ene dihydrobromide and 299mg of 6,7,8-trifluoro-1-(t-butyl)-1,4-dihydro-4-oxoquinoline-3-carboxylic acid were subjected to the same process as described in Example 1 to obtain 238mg of the desired compound (yield: 57%).

| | |
|---|---|
| m.p.: | 205~ 208 °C |
| $^1$H-NMR ($CDCl_3$ + $CD_3COOD$, δ ppm) ; | 8.56 (1H, s), 8.15 (1H, d, J = 14.2 Hz), 4.50~4.30 (5H, br, s), 2.93 (1H, m), 2.71 (3H, s), 2.62~2.43 (2H, m), 2.30 (1H, m), 1.81 (9H, s) |

Preparation-

Example 26: Preparation of 7-[6-amino-3-azabicyclo[3,3,0]oct-1(5) ene-3-yl]-1-(t-butyl)-6-fluoro-8-chloro-1,4-dihydro-4-oxoquinoline-3-carboxylic acid

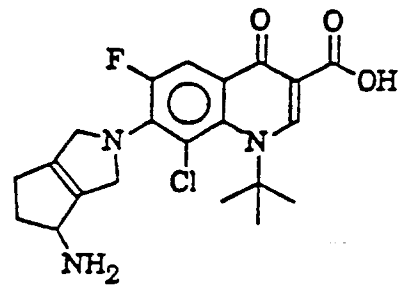

325mg of 6-amino-3-azabicyclo[3,3,0]oct-1(5)ene dihydrobromide and 315mg of 6,7-difluoro-8-chloro-1-(t-butyl)-1,4-dihydro-4-oxoquinoline-3-carboxylic acid were subjected to the same process as described in Example 1 to obtain 206mg of the desired compound (yield: 49%).

| | |
|---|---|
| m.p.: | 179~ 184 °C |
| $^1$H-NMR ($CDCl_3$ + $CD_3COOD$, δ ppm) ; | 8.55 (1H, s), 8.09 (1H, d, J = 14.2 Hz), 4.51~4.32 (5H, m), 2.95 (1H, m), 2.65~2.42 (2H, m), 2.25 (1H, m), 1.85 (9H, s) |

Preparation-

Example 27: Preparation of 7-[6-methylamino-3-azabicyclo[3,3,0]oct-1(5)ene-3-yl]-1-(t-butyl)-6-fluoro-8-chloro-1,4-dihydro-4-oxoquinoline-3-carboxylic acid

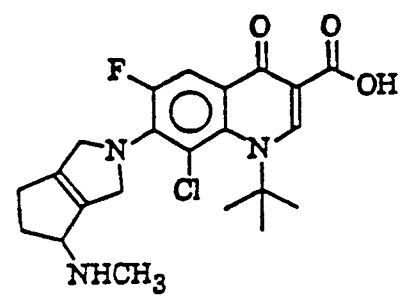

355mg of 6-methylamino-3-azabicyclo[3,3,0]oct-1(5)ene dihydrobromide and 315mg of 6,7-difluoro-8-chloro-1-(t-butyl)-1,4-dihydro-4-oxoquinoline-3-carboxylic acid were subejcted to the same process as described in Example 1 to obtain 195mg of the desired compound(yield: 45%).

| | |
|---|---|
| m.p.: | 175~ 178 °C |
| $^1$H-NMR ($CDCl_3$ + $CD_3COOD$, δ ppm) ; | 8.54 (1H, s), 8.05 (1H, d, J = 14.2 Hz), 4.55~4.30 (5H, m), 2.95 (1H, m), 2.72 (3H, s), 2.65~2.45 (2H, m), 2.24 (1H, m), 1.84 (9H, s) |

Preparation-

Example 28: Preparation of 7-[6-amino-3-azabicyclo[3,3,0]oct-1(5) ene-3-yl]-1-(4-fluorophenyl)-6,8-difluoro-1,4-dihydro-4-oxoquinoline-3-carboxylic acid

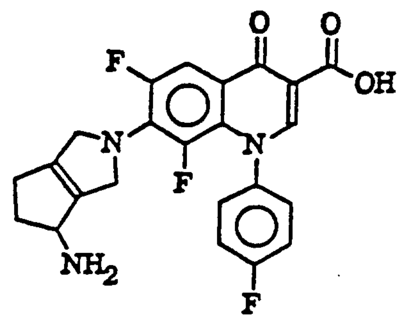

325mg of 6-amino-3-azabicyclo[3,3,0]oct-1(5)ene dihydrobromide and 337mg of 1-(4-fluorophenyl)-6,7,8-trifluoro-1,4-dihydro-4-oxoquinoline-3-carboxylic acid were subjected to the same process as described in Example 1 to obtain 274mg of the desired compound (yield: 62%).

| | |
|---|---|
| m.p.: | 221~ 224°C |
| $^1$H-NMR ($CDCl_3$ + $CD_3COOD$, δ ppm) ; | 8.59 (1H, s), 8.01 (1H, d, J = 14.2 Hz), 7.49 (2H, m), 7.25 (2H, m), 4.75~4.49 (5H, m), 2.91~2.37 (4H, m) |

Preparation-

Example 29: Preparation of 7-[6-methylamino-3-azabicyclo[3,3,0]oct-1(5)ene-3-yl]-1-(4-fluorophenyl)-6,8-difluoro-1,4-dihydro-4-oxoquinoline-3-carboxylic acid

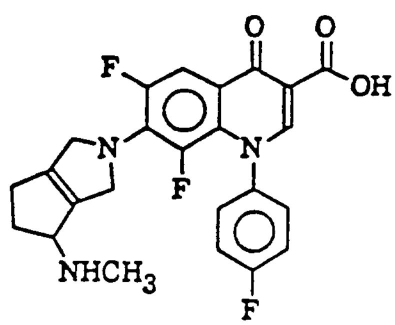

355mg of 6-methylamino-3-azabicyclo[3,3,0]oct-1(5)ene dihydrobromide and 337mg of 1-(4-fluorophenyl)-6,7,8-trifluoro-1,4-dihydro-4-oxoquinoline-3-carboxylic acid were subejcted to the same process as described in Example 1 to obtain 296mg of the desired compound(yield: 65%).

| | |
|---|---|
| m.p.: | 227~ 230°C |
| $^1$H-NMR ($CDCl_3$ + $CD_3COOD$, δ ppm) ; | 8.57 (1H, s), 7.99 (1H, d, J = 14.2 Hz), 7.48 (2H, m), 7.26 (2H, m), 4.76~4.51 (5H, m), 2.73 (3H, s), 2.90~2.36 (4H, m) |

Preparation-

Example 30: Preparation of 7-[6-amino-3-azabicyclo[3,3,0]oct-1(5) ene-3-yl]-1-ethyl-6-fluoro-1,4-dihydro-4-oxo-1,8-naphthyridine-3-carboxylic acid

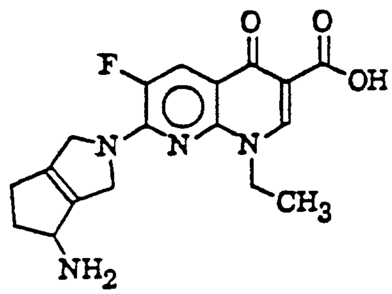

325mg of 6-amino-3-azabicyclo[3,3,0]oct-1(5)ene dihydrobromide and 270mg of 1-ethyl-7-chloro-6-fluoro-4-oxo-1,8-naphthyridine-3-carboxylic acid were subjected to the same process as described in Example 1 to obtain 205mg of the desired compound (yield: 57%).

| | |
|---|---|
| m.p: | 179~ 183 °C |
| $^1$H-NMR ($CDCl_3$ + $CD_3COOD$, δ ppm) ; | 8.75 (1H, s), 8.09 (1H, d, J = 12.4 Hz), 4.75~4.35 (7H, m), 2.90~2.45 (4H, m), 1.51 (3H, t, J = 7 Hz) |

Preparation-

Example 31: Preparation of 7-[6-methylamino-3-azabicyclo[3,3,0]oct-1(5)ene-3-yl]-1-ethyl-6-fluoro-1,4-dihydro-4-oxo-1,8-naphthyridine-3-carboxylic acid

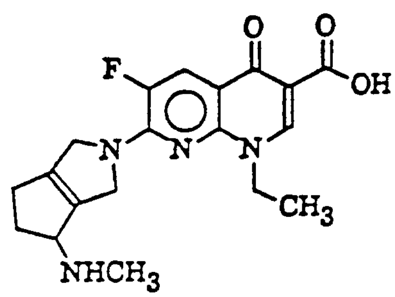

355mg of 6-methylamino-3-azabicyclo[3,3,0]oct-1(5)ene dihydrobromide and 270mg of 1-ethyl-7-chloro-6-fluoro-4-oxo-1,8-naphthyridine-3-carboxylic acid were subjected to the same process as described in Example 1 to obtain 231mg of the desired compound (yield: 62%).

| | |
|---|---|
| m.p.: | 185~ 189 °C |
| $^1$H-NMR ($CDCl_3$ + $CD_3COOD$, δ ppm) ; | 8.74 (1H, s), 8.07 (1H, d, J = 12.4 Hz), 4.76~4.36 (7H, m), 2.71 (3H, s), 2.90~2.44 (4H, m), 1.51 (3H, t, J = 7 Hz) |

Preparation-

Example 32: Preparation of 7-[6-amino-3-azabicyclo[3,3,0]oct-1(5) ene-3-yl]-1-(t-butyl)-6-fluoro-1,4-dihydro-4-oxoquinoline-3-carboxylic acid

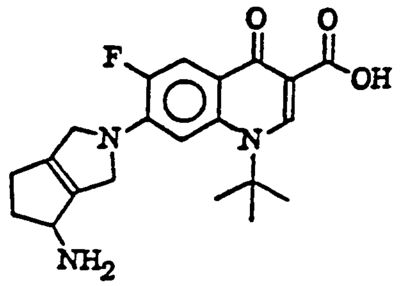

320mg of 6-amino-3-azabicyclo[3,3,0]oct-1(5)ene dihydrobromide and 281mg of 1-(t-butyl)-6,7-difluoro-1,4-dihydro-4-oxoquinoline-3-carboxylic acid were subejcted to the same process as described in Example 1 to obtain 205mg of the desired compound (yield: 53%).

| | |
|---|---|
| m.p.: | 193~ 196 °C |
| $^1$H-NMR ($CDCl_3$ + $CD_3COOD$, δ ppm) ; | 8.55 (1H, s), 7.83 (1H, d, J = 12.3 Hz), 7.21 (1H, s), 4.70~4.49 (5H, m), 2.85~2.43 (4H, m), 1.91 (9H, s) |

Preparation-

Example 33: Preparation of 7-[6-methylamino-3-azabicyclo[3,3,0]oct-1(5)ene-3-yl]-1-(t-butyl)-6-fluoro-1,4-dihydro-4-oxoquinoline-3-carboxylic acid

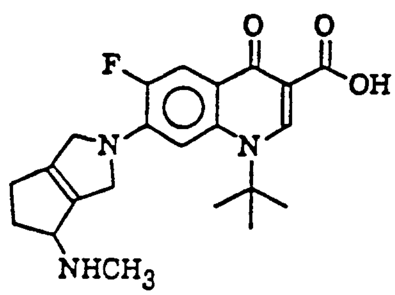

350mg of 6-methylamino-3-azabicyclo[3,3,0]oct-1(5)ene dihydrobromide and 281mg of 1-(t-butyl)-6,7-difluoro-1,4-dihydro-4-oxoquinoline-3-carboxylic acid were subjected to the same process as described in Example 1 to obtain 248mg of the desired compound (yield: 62%).

| | |
|---|---|
| m.p.: | 198~ 202 °C |
| $^1$H-NMR ($CDCl_3$ + $CD_3COOD$, δ ppm) ; | 8.82 (1H, s),7.81 (1H, d, J = 12.3 Hz),7.19 (1H, s), 4.70~4.48 (5H, m), 2.85~2.40 (4H, m), 2.72 (3H, s), 1.90 (9H, s) |

Preparation-

Example 34: Preparation of 7-[6-acetamino-3-azabicyclo[3,3,0]oct-1(5)ene-3-yl]-1-cyclopropyl-6,8-difluoro-1,4-dihydro-4-oxoquinoline-3-carboxylic acid

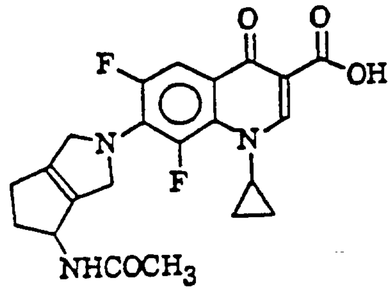

193mg of 7-[6-amino-3-azabicyclo[3,3,0]oct-1(5)ene-3-yl]-1-cyclopropyl-6,8-difluoro-1,4-dihydro-4-oxoquinoline-3-carboxylic acid was dissolved in a mixture of 3mℓ of pyridine and 2.5mℓ of acetic anhydride; and the resulting solution was stirred at a room temperature over night. The reaction mixture was poured into 20mℓ of water and stirred at a room temperature for 2 hours. The precipitate produced was filtered, washed with water, isopropanol and ethylether and dried under a reduced pressure to obtain 174mg of the desired compound(yield: 90%).

| | |
|---|---|
| m.p.: | 199~ 203 °C |
| $^1$H-NMR ($CDCl_3$ + $CD_3COOD$, δ ppm) ; | 8.77 (1H, s), 7.78 (1H, d, J = 14.6 Hz), 4.60 (4H, br, s), 4.50 (1H, m), 4.05 (1H, m), 2.91 (1H, m), 2.60 (1H, m), 2.52 (1H, m), 2.35 (1H, m), 2.01 (3H, s), 1.26 (4H, m) |

Preparation-

Example 35: Preparation of 7-[6-t-butoxycarbonylamino-3-azabicyclo-[3,3,0]oct-1(5)ene-3-yl]-1-cyclopropyl-6,8-difluoro-1,4-dihydro-4-oxoquinoline-3-carboxylic acid methoxymethylester

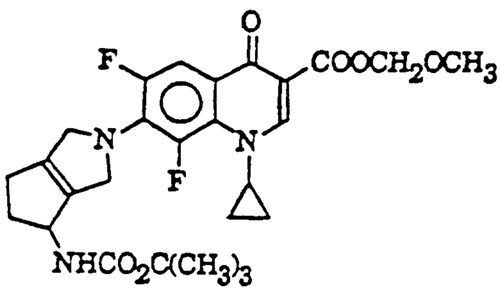

487mg of 7-[6-t-butoxycarbonylamino-3-azabicyclo[3,3,0] oct-1(5)ene-3-yl]-1-cyclopropyl-6,8-difluoro-1,4-dihydro-4-oxoquinoline-3-carboxylic acid was suspended in a mixture of 10mℓ of hexamethylphosphoramide and 5mℓ of tetrahydrofuran. To the resulting solution were added 250mg of methoxymethyliodide and 200mg of potassium carbonate powder; and the reaction mixture was stirred at a room temperature for 24 hours followed by an addition of 50mℓ of dichloromethane. The resultant was washed with water and 5% $NaHCO_3$; and the organic layer was then dehydrated with anhydrous sodium sulfate and concentrated, The residue thus obtained was crystallized with chloroform-hexane to obtain 298mg of the desired compound (yield: 56%).

| | |
|---|---|
| m.p.: | 155~ 158 °C |
| $^1$H-NMR ($CDCl_3$, δ ppm) ; | 8.79 (1H, s), 7.75 (1H, d, J = 14.5 Hz), 4.62 (4H, br, s), 4.51 (1H, m), 4.25 (2H, br, s), 4.06 (1H, m), 3.81 (3H, s), 2.90 (1H, m), 2.61 (1H, m), 2.54 (1H, m), 2.33 (1H, m), 1.92 (9H, s), 1.25~1.15 (4H, m) |

Preparation-

Example 36: Preparation of 7-[6-amino-3-azabicyclo[3,3,0]oct-1(5) ene-3-yl]-6,8-difluoro-1,4-dihydro-4-oxoquinoline-3-carboxylic acid lactate

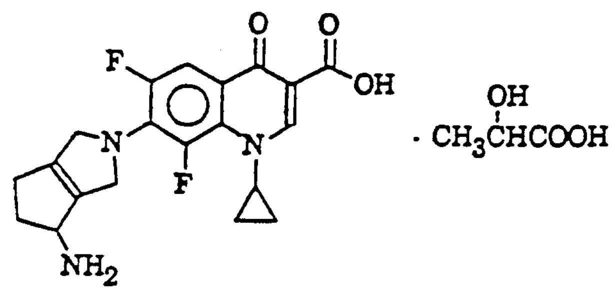

387mg of 7-[6-amino-3-azabicyclo[3,3,0]oct-1(5)ene-3-yl]-6,8-difluoro-1,4-dihydro-4-oxoquinoline-3-carboxylic acid was dissolved in a mixture of 10mℓ of dichloromethane and 2mℓ of ethanol; and the resulting solution was stirred at a room temperature for 2 hours after an addition of 87μℓ of 85% lactic acid. The solvent was evaporated under a reduced pressure to obtain quantitatively the desired compound.

| | |
|---|---|
| m.p.: | 180~ 184 °C |
| $^1$H-NMR ($CD_3OD + CD_3COOD$, δ ppm) ; | 8.74 (1H, s), 7.72 (1H, d, J = 14 Hz), 4.8~4.1 (7H, m), 2.85~2.35 (4H, m), 1.50~1.16 (7H, m) |

Use Example 1

A capsule formulation was prepared in accordance with the following composition:

| Component | Amount |
|---|---|
| Compound prepared in Example 1 | 100.0mg |
| corn starch | 25.0mg |
| calcium carboxymethyl cellulose | 23.0mg |
| magnesium stearate | 2.0mg |
| total | 150.0mg |

Use Example 2

A solution formulation was prepared in accordance with the following composition:

| Component | Amount |
|---|---|
| Compound prepared in Example 36 | 1 to 10g |
| lactic acid or sodium hydroxide | 0.1 to 2g |
| mannitol | 0.1g |
| deionized water | 87.9 to 98.8g |
| total | 150g |

1. In vitro anti-bacterial activity test

In order to demonstrate the superior effectiveness of the quinolone derivatives of the present invention, the minimal inhibitory concentration(MIC) of several compounds synthesized in Examples hereof against the standard strains was determined and compared with ofloxacin and ciprofloxacin. These MIC values were

taken by employing two-fold dilution method: that is, two-fold serial dilutions of each of the test compounds were made and dispersed in a Mueller-Hinton agar medium; a standard strain which had the value of $10^7$ CFU/mℓ was inoculated on the medium, which was then incubated at 37°C for 18 hours. The results of the MIC tests are shown in Table 1.

Table 1. *In vitro* anti-bacterial activity test(MIC: μg/mℓ)

| No | Strain | Compound prepared in Example 1 | Compound prepared in Example 2 | Compound prepared in Example 3 | Compound prepared in Example 4 |
|---|---|---|---|---|---|
| 1 | Streptococcus pyogenes 308A | 0.391 | 0.195 | 0.195 | 0.195 |
| 2 | Streptococcus pyogenes 77A | 0.098 | 0.098 | 0.049 | 0.025 |
| 3 | Streptococcus faecium MD 8b | 0.098 | 0.098 | 0.098 | 0.049 |
| 4 | Staphylococcus aureus SG 511 | 0.098 | 0.049 | 0.025 | 0.025 |
| 5 | Staphylococcus aureus 285 | 0.098 | 0.098 | 0.049 | 0.049 |
| 6 | Staphylococcus aureus 503 | 0.049 | 0.049 | 0.049 | 0.025 |
| 7 | Escherichia coli O 55 | 0.025 | 0.013 | 0.049 | 0.025 |
| 8 | Escherichia coli DC 0 | 0.391 | 0.781 | 0.781 | 0.781 |
| 9 | Escherichia coli DC 2 | 0.098 | 0.098 | 0.049 | 0.025 |
| 10 | Escherichia coli OTEM | 0.049 | 0.098 | 0.098 | 0.098 |
| 11 | Escherichia coli 1507E | 0.049 | 0.098 | 0.098 | 0.098 |
| 12 | Pseudomonas aeruginosa 9027 | 1.563 | 3.125 | 1.563 | 3.125 |
| 13 | Pseudomonas aeruginosa 1592E | 1.563 | 1.563 | 1.563 | 3.125 |
| 14 | Pseudomonas aeruginosa 1771 | 1.563 | 1.563 | 1.563 | 3.125 |
| 15 | Pseudomonas aeruginosa 1771M | 0.391 | 0.781 | 0.391 | 0.781 |
| 16 | Salmonella typhimurium | 0.049 | 0.391 | 0.049 | 0.049 |
| 17 | Klebsiella aerogenes 1082E | 0.007 | 0.013 | 0.013 | <0.002 |
| 18 | Klebsiella aerogenes 1522E | 0.098 | 0.049 | 0.195 | 0.195 |
| 19 | Enterobacter cloacae P99 | 0.098 | 0.049 | 0.098 | 0.098 |
| 20 | Enterobacter cloacae 1321E | 0.025 | 0.013 | 0.098 | 0.098 |

Table 1(Continued)

| No | Strain | Compound prepared in Example 9 | Compound prepared in Example 10 | Compound prepared in Example 11 | Compound prepared in Example 15 |
|---|---|---|---|---|---|
| 1 | Streptococcus pyogenes 308A | 0.391 | 0.195 | 0.391 | 0.781 |
| 2 | Streptococcus pyogenes 77A | 0.098 | 0.049 | 0.195 | 0.195 |
| 3 | Streptococcus faecium MD 8b | 0.098 | 0.195 | 0.195 | 0.195 |
| 4 | Staphylococcus aureus SG 511 | 0.098 | 0.025 | 0.195 | 0.098 |
| 5 | Staphylococcus aureus 285 | 0.098 | 0.049 | 0.049 | 0.195 |
| 6 | Staphylococcus aureus 503 | 0.098 | 0.025 | 0.049 | 0.098 |
| 7 | Escherichia coli O 55 | 0.049 | 0.025 | 0.025 | 0.049 |
| 8 | Escherichia coli DC 0 | 0.781 | 0.781 | 1.563 | 1.563 |
| 9 | Escherichia coli DC 2 | 0.195 | 0.049 | 0.049 | 0.391 |
| 10 | Escherichia coli OTEM | 0.098 | 0.049 | 0.049 | 0.098 |
| 11 | Escherichia coli 1507E | 0.098 | 0.049 | 0.098 | 0.195 |
| 12 | Pseudomonas aeruginosa 9027 | 3.125 | 1.563 | 3.125 | 3.125 |
| 13 | Pseudomonas aeruginosa 1592E | 3.125 | 1.563 | 3.125 | 3.125 |
| 14 | Pseudomonas aeruginosa 1771 | 3.125 | 1.563 | 3.125 | 3.125 |
| 15 | Pseudomonas aeruginosa 1771M | 0.781 | 0.781 | 1.563 | 1.563 |
| 16 | Salmonella typhimurium | 0.013 | 0.025 | 0.098 | 0.195 |
| 17 | Klebsiella aerogenes 1082E | 0.195 | 0.007 | 0.007 | 0.025 |
| 18 | Klebsiella aerogenes 1522E | 0.195 | 0.098 | 0.195 | 0.195 |
| 19 | Enterobacter cloacae P99 | 0.049 | 0.025 | 0.195 | 0.049 |
| 20 | Enterobacter cloacae 1321E |  | 0.025 | 0.049 | 0.049 |

Table 1(Continued)

| No | Strain | Compound prepared in Example 17 | Ofloxacin | Cipro-floxacin |
|---|---|---|---|---|
| 1 | Streptococcus pyogenes 308A | 3.125 | 3.125 | 3.125 |
| 2 | Streptococcus pyogenes 77A | 0.195 | 1.563 | 0.781 |
| 3 | Streptococcus faecium MD 8b | 0.391 | 0.781 | 0.781 |
| 4 | Staphylococcus aureus SG 511 | 0.391 | 0.391 | 0.781 |
| 5 | Staphylococcus aureus 285 | 0.781 | 0.391 | 0.781 |
| 6 | Staphylococcus aureus 503 | 0.781 | 0.391 | 0.781 |
| 7 | Escherichia coli O 55 | 0.195 | 0.025 | 0.007 |
| 8 | Escherichia coli DC 0 | 3.125 | 0.781 | 0.391 |
| 9 | Escherichia coli DC 2 | 0.391 | 0.391 | 0.195 |
| 10 | Escherichia coli OTEM | 0.391 | 0.049 | 0.013 |
| 11 | Escherichia coli 1507E | 0.781 | 0.098 | 0.025 |
| 12 | Pseudomonas aeruginosa 9027 | 12.500 | 1.563 | 0.781 |
| 13 | Pseudomonas aeruginosa 1592E | 6.250 | 1.563 | 0.391 |
| 14 | Pseudomonas aeruginosa 1771 | 12.500 | 1.563 | 0.781 |
| 15 | Pseudomonas aeruginosa 1771M | 3.125 | 0.391 | 0.195 |
| 16 | Salmonella typhimurium | 0.391 | 0.098 | 0.049 |
| 17 | Klebsiella aerogenes 1082E | 0.195 | 0.025 | 0.025 |
| 18 | Klebsiella aerogenes 1522E | 1.563 | 0.098 | 0.013 |
| 19 | Enterobacter cloacae P99 | 0.781 | 0.098 | 0.025 |
| 20 | Enterobacter cloacae 1321E | 0.391 | 0.049 | 0.007 |

2. Preventive effect on the systemic infection

10 fold of the pathogen which leads to 100% lethality were injected intraperitoneally to male and female NMRI mice weighing 18 to 20g. Immediately and after 4 hours, the dose of test compounds which was determined by two-fold serial dilution method was administered orally or subcutaneously; and on day 7, the effectiveness was evaluated in terms of $ED_{50}$ calculated from the number of survived mice by the probit analysis.

Table 2

| Preventive effect on the systemic infection | | | |
|---|---|---|---|
| Pathogen | Compound (Preparation-Examples) | $ED_{50}$: mg/kg | |
| | | Subcutaneous Injection | Oral Administration |
| S. aureus 17740 | Compound prepared in Example 1 | 25 | 105 |
| | Compound prepared in Example 2 | 27 | 112 |
| | Ofloxacin<br>Ciprofloxacin | 200.0<br>132.3 | >200.0<br>>200.0 |
| P. mirabilis | Compound prepared in Example 1<br>Compound prepared in Example 3<br>Ofloxacin<br>Ciprofloxacin | 1.55<br>1.43<br>0.53<br>0.28 | 5.5<br>5.9<br>2.27<br>0.92 |

3. Acute Toxicity Test

Test compounds were administered to female ICR mice weighing 20 to 25g and on day 14, $LD_{50}$ was calculated from the number of survived mice by the probit analysis.

Table 3

| Result of the Acute Toxicity Test | | |
|---|---|---|
| Compound (Preparation Exampl.) | Acute Toxicity($LD_{50}$: mg/kg) | |
| | Intraperitoneal Injection | Oral Administration |
| Compound prepared in Example 1 | 720 | 4000 |
| Compound prepared in Example 2 | 810 | 4100 |
| Compound prepared in Example 3 | 731 | 4100 |
| Compound prepared in Example 6 | 755 | 4300 |

As can be seen from the results, the compounds of the present invention possess a broad spectrum of potent anti-bacterial activities against gram-positive and-negative bacteria as compared with the known quinolone antibiotics, ciprofloxacin and ofloxacin. The compounds of the present invention also exhibit superior activities to the known quinolone antibiotics in terms of 50% effective dose($ED_{50}$) on the systemic bacterial infection. Further, the compounds of the present invention have low toxicity sufficient to be proved as drugs.

Accordingly, the compounds of the present invention will be useful as therapeutical compounds and preservatives of inorganic and organic material.

**Claims**

**1.** A quinolone carboxylic acid derivative of formula(I) and pharmaceutically acceptable salts thereof:

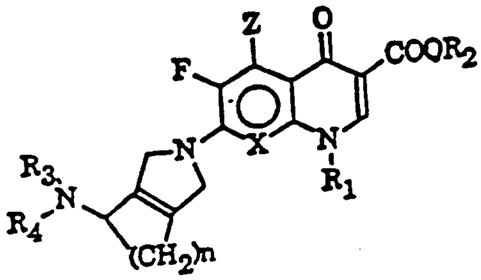

(I)

wherein:

| | |
|---|---|
| X is | a nitrogen atom or a C-Y group wherein Y is a hydrogen, fluorine, chlorine or bromine atom or a methoxy or methyl group; |
| $R_1$ is | a $C_{1-6}$ alkyl group optionally substituted with a halogen atom or a hydroxy radical, an alkenyl group, a $C_{3-6}$ cycloalkyl group, a phenyl group substituted with a halogen atom or a divalent group of $-OCH_2{}^*CH(CH_3)-$, $-SCH_2{}^*CH_2-$or $-SCH_2{}^*CH(CH_3)-$ which forms an oxazine or thiazine ring together with the nitrogen atom to which $R_1$ is attached and with X wherein X is C-Y; |
| $R_2$ is | a hydrogen atom, a carboxy protecting group or a pharmaceutically acceptable metal or organic cation; |
| $R_3$ and $R_4$, | which may be the same or different, are a hydrogen atom, a lower alkyl group, an acyl group or a nitrogen protecting group metabolizable in vivo; |
| Z is | a hydrogen or halogen atom, an amino, hydroxy or methyl group; and |
| n is | 1 to 3. |

**2.** The compound of claim 1 wherein a carboxy protecting group is a lower alkyl, lower alkanoyloxy-lower alkyl, lower alkoxycarbonyloxy-lower alkyl, lower alkoxymethyl, di(lower alkyl) amino-lower alkyl or 4-methylene-5-methyl-1,3-dioxalene-2-one group.

**3.** The compound of claim 1 which is the salt with a hydrochloric, sulfuric, phosphoric, nitric, methanesulfonic, acetic, formic, propionic, lactic, maleic, malonic, fumaric, tataric, citric, paratoluenesulfonic, ascorbic or glutamic acid.

**4.** A process for preparing a compound of formula(Ia), which comprises reacting a compound of formula(II) with a compound of formula(III) or (IIIa)

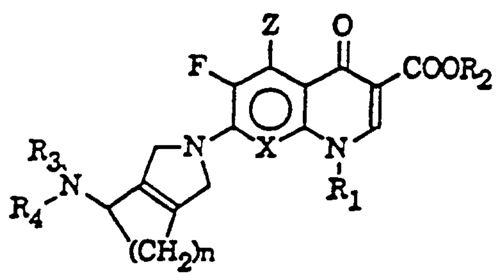

(Ia)

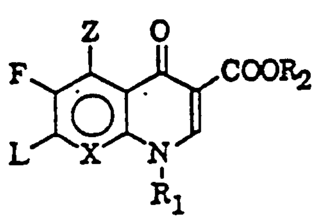

(II)

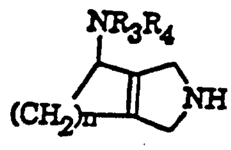

(III)

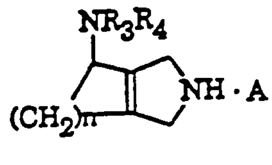

(IIIa)

whrein:

| | |
|---|---|
| $R_1$, $R_3$, $R_4$, X, Z and n | are the same as defined in claim 1; |
| $R_2$ is | a hydrogen atom or a carboxy protecting group as defined in claim 2; |
| L is | a leaving group which is a fluorine, chlorine or bromine atom, a methanesulfonyl group or a para-toluenesulfonyl group; and |
| A is | a hydrochloric, bromic, sulfuric or trifluoroacetic acid. |

**5.** A process for preparing a compound of formula(Ia-2) wherein $R_2$ is carboxy protecting group, which comprises reacting a compound of formula(Ia-1) with a compound of $R_2$-Hal:

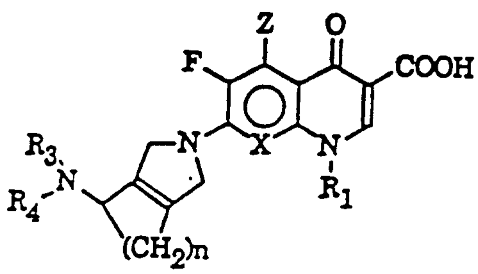

(Ia-1)

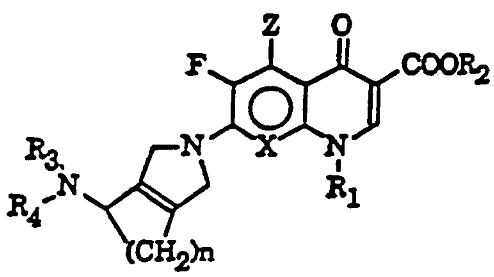

(Ia-2)

wherein:

| | |
|---|---|
| $R_1$, $R_3$, $R_4$, X, Z and n | are the same as defined in claim 1; |
| $R_2$ is | a carboxy protecting group as defined in claim 2; and |
| Hal is | a chlorine, bromine or iodine atom. |

**6.** A process for preparing a compound of formula(Ia-3) wherein $R_2$ is a pharmaceutically acceptable metal or organic cation, which comprises reacting a compound of formula(Ia-1) with a compound of $R_2$-donor:

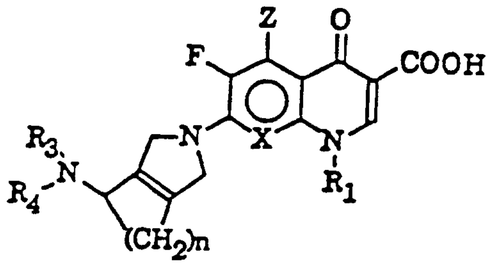

(Ia-1)

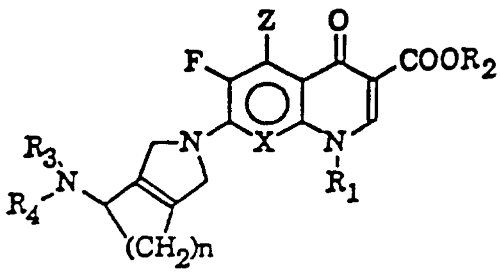

(Ia-3)

wherein:

| | |
|---|---|
| $R_1$, $R_3$, $R_4$, X, Z and n | are the same as defined in claim 1; and |
| $R_2$-donor | is sodium hydroxide, potassium hydroxide, calcium hydroxide, magnesium hydroxide, silver nitrate or tertiary or quarternary $C_{1-4}$ alkylammonium. |

7. A process for preparing a compound of formula(Ia-5), which comprises reacting a compound of formula(Ia-4) with a compound of $R_4$-donor: wherein:

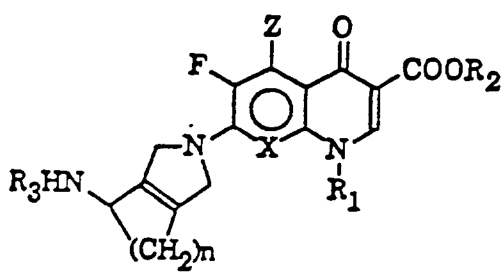

(Ia-4)

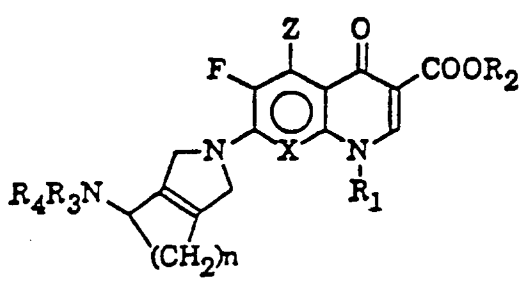

(Ia-5)

| | |
|---|---|
| $R_1$, $R_3$, $R_4$, X, Z and n | are the same as defined in claim 1; |
| $R_2$ is | a hydrogen atom or a carboxy protecting group as defined in claim 2; and |
| $R_4$-donor | is $R_4$-Hal wherein Hal is a chlorine, bromine or iodine atom and $R_4$ is the same as defined in claim 1, excepting a hydrogen atom. |

8. An antibacterial formulation comprising at least one of the compounds of formula(I) as an active component.

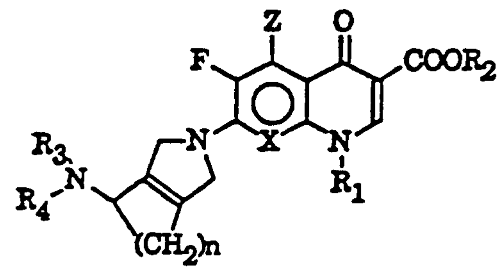

(I)

wherein:

R₁, R₂, R₃, R₄, X, Z and n are the same as defined in claim 1.

**9.** An azabicycloamine of formula (I) and the salts thereof:

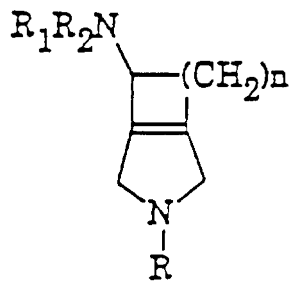

(I)

wherein

| | |
|---|---|
| R is | hydrogen or an amine protecting group; |
| $R_1$ and $R_2$, | which may be the same or different, are hydrogen, optionally substituted lower alkyl, alkanoyl, optionally substituted benzoyl or naphthoyl, optionally substituted alkoxycarbonyl, or optionally substituted benzyloxycarbonyl; and |
| n is | an integer from 1 to 4. |

**10.** An azabicycloamine of claim 9 and the salts thereof, wherein the amine protecting group in R is benzenesulfonyl, paratoluenesulfonyl, methanesulfonyl, $C_{1-4}$ alkoxycarbonyl, benzyloxycarbonyl, para-nitrobenzyloxycarbonyl, 2,2,2-trichlorethoxycarbonyl, $C_{1-20}$ alkanoyl, or optionally substituted benzoyl or naphthoyl.

**11.** A compound of claim 9 wherein R, $R_1$ and $R_2$ are hydrogen, as represented by the formula (I-1), and the acid addition salts thereof as represented by formula (Ia-1):

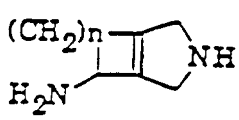

(I-1)

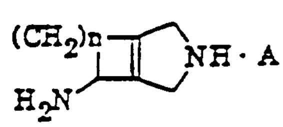

(Ia-1)

wherein A is a hydrochloric acid, hydrobromic acid, sulfuric acid or trifluoroacetic acid.

**12.** A compound of claim 9 wherein R and $R_1$ are hydrogen atoms; and $R_2$ is optionally substituted lower alkyl, alkanoyl, optionally substituted benzoyl or naphthoyl, optionally substituted alkoxycarbonyl, or optionally substituted benzyloxycarbonyl, as represented by formula (I-2), and the addition salts thereof as represented by formula (Ia-2):

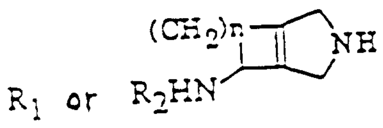

(I-2)

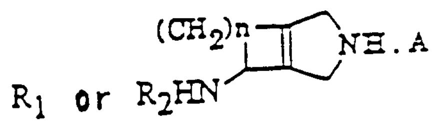

(Ia-2)

wherein A is hydrochloric, hydrobromic, sulfuric or trifluoroacetic acid.

**13.** A compound of claim 9 and the salts thereof wherein n is 2 or 3.

**14.** A salt of claim 9 which is an acid addition salt, wherein the acid of the acid addition salt is an inorganic acid selected from the group consisting of hydrochloric, hydrobromic, sulfuric and phosphoric acid, or an organic acid selected from the group consisting of formic, acetic, propionic, methane sulfonic, trichloroacetic, trifluoroacetic, lactic, maleic, malonic, fumaric, citric, tartaric, paratoluenesulfonic, benzenesulfonic, palmitic, oleic and stearic acid.

**15.** A process for preparing a compound of formula (Ia-1) of claim 11,which comprises:

(a) heating a compound of formula (II) with N-bromo (or chloro) succinimide in the presence of a free radical-generating agent to obtain a compound of formula (III);

(b) reacting the compound of formula (III) with an azide to obtain a compound of formula (IV);

(c) reacting the compound of formula (IV) with triphenylphosphine in a solvent to obtain a compound of formula (V);

(d) reacting the compound of the formula (V) with an amine protecting agent to obtain a compound of formula (VI);

(e) reducing the compound of formula (VI) with a reducing agent to obtain a compound of formula (VII);

(f) reacting the compound of the formula (VII) with a halogenation agent selected from the group consisting of phosphorous trichloride, phosphorous trifluoride, thionylchloride and oxalylchloride; or with triphenylphosphine and with carbon tetrachloride or carbon tetrabromide to obtain a compound of the formula (VIII);

(g) reacting the compound of formula (VIII) with an $R_4$ donating agent selected from the group consisting of paratoluenesulfonamide, benzenesulfonamide. acetamide, benzamide, methanesulfonamide and urethane in the presence of a base to obtain a compound of formula (IX); and

(h) heating the compound of formula (IX) in an acid selected from the group consisting of hydrochloric, sulfuric hydrobromic and trifluoroacetic acid:

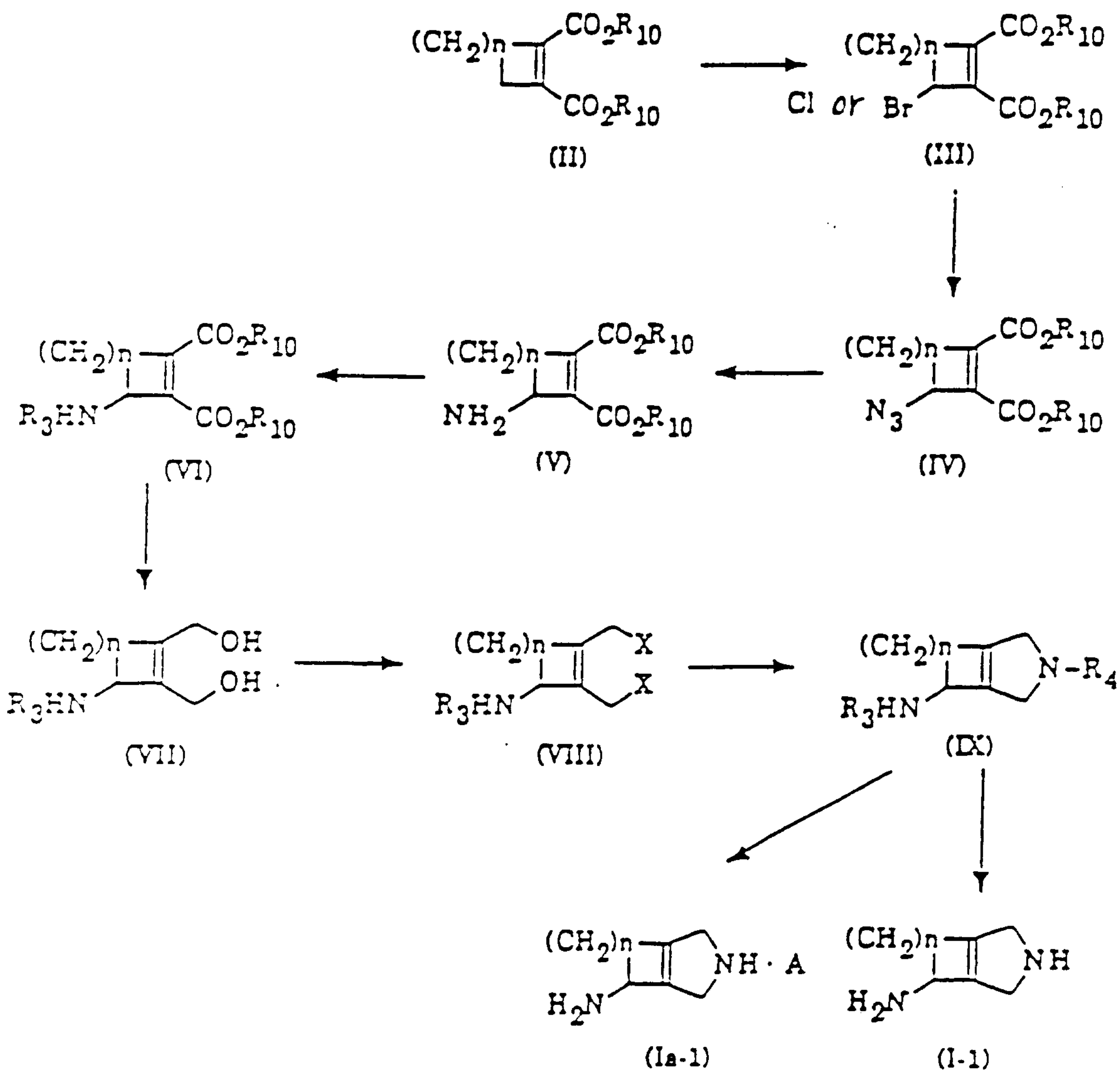


wherein:

n is an integer from 1 to 4;
$R_{10}$ is a $C_{1-4}$ alkyl;
$R_3$ is an amine protecting group;
$R_4$ is a hydrogen atom or an amino protecting group;
X is an iodine, bromine or chlorine atom, or O-mesyl or O-tosyl group; and
A is hydrochloric, hydrobromic, sulfonic or trifluoroacetic acid.

**16.** The process of claim 15,wherein $R_3$ is methoxycarbonyl, ethoxycarbonyl, t-butoxycarbonyl, 2,2,2-trichloroethoxycarbonyl, benzyloxycarbonyl and paranitrobenzyloxycarbonyl.

**17.** The process of claim 15,wherein the amino protecting group is benzenesulfonyl, paratoluene sulfonyl, methanesulfonyl or lower alkoxycarbonyl.

**18.** The process of claim 15,wherein the free radical-generating agent is benzoylperoxide or azaisobutyronitrile.

**19.** The process of claim 15,wherein the amine protecting agent is selected from the group consisting of di-t-butyl dicarbonate or methyl-, ethyl-, trichloromethyl-, benzyl- or paranitrobenzyl chloroformate in the presence of pyridine, sodium hydride, potassium carbonate, triethyl amine, sodium hydroxide.

**20.** A process for preparing a compound of formula (I-1), which comprises removing the nitrogen protecting group in the presence of a base.

**21.** The process of claim 20, wherein the base is sodium hydroxide or sodium ammonia.

**22.** A process for preparing a compound of formula (I-2) or (Ia-2), which comprises:
(a) reacting a compound of formula (III) with sodium acetate or silver acetate to obtain a compound of formula (X);
(b) deacetylating the compound of formula (X) to obtain a compound of formula (XI);
(c) mesylating or tosylating the compound of formula (XI) and reacting with a $R_2$ donating amine compound; or reacting the compound of formula (XI) by employing Mitsunobu reaction to protect the amine in a compound of the formula (XII); and
(d) following the same procedures as in steps of (e), (f), (g) and (h) of claim 7:

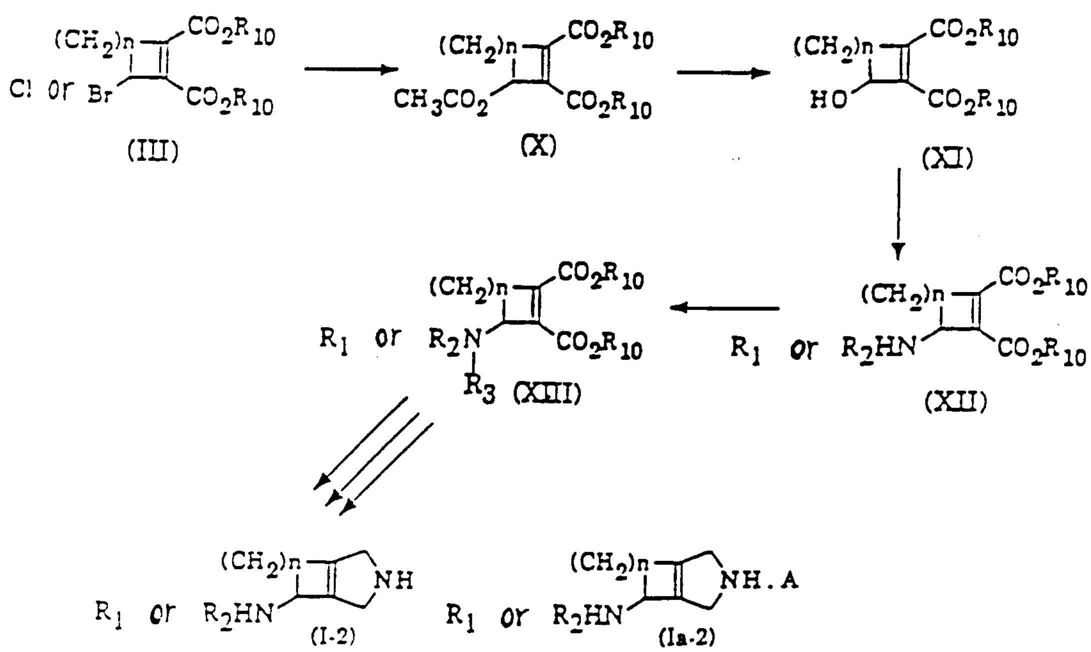

wherein
n, R, $R_2$, $R_3$ and A have the same meanings as defined above.

**23.** A process for preparing a compound of formula (I-3) or (Ia-3), which comprises reacting a compound of formula (IX) with an alkylhalide in the presence of a strong base to obtain a compound of formula (XIV) from which $R_2$ and $R_4$ the amino protecting groups, are removed to produce the compound of formula (I-3) or (Ia-3)

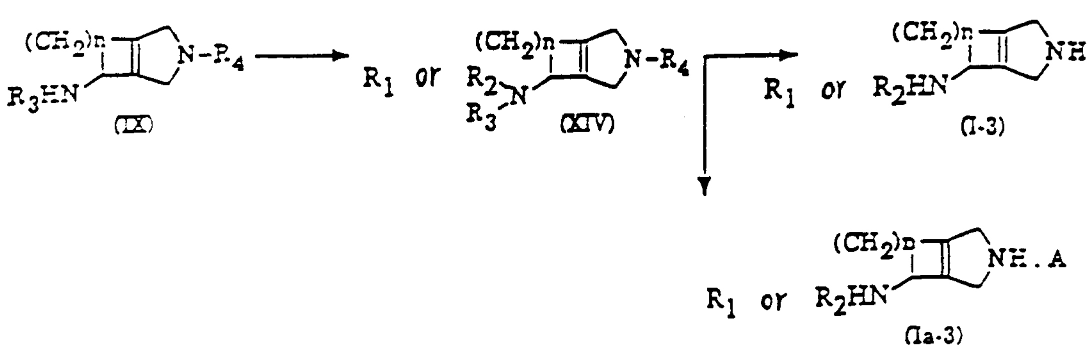

wherein n, $R_2$, $R_3$, $R_4$ and A are as defined above.

**24.** A process for preparing a compound of formula (I-4) or (Ia-4), which comprises reducing a compound of formula (XV) in the presence of aluminum lithium hydride to obtain a compound of formula (XVI) and removing the protecting group $R_6$

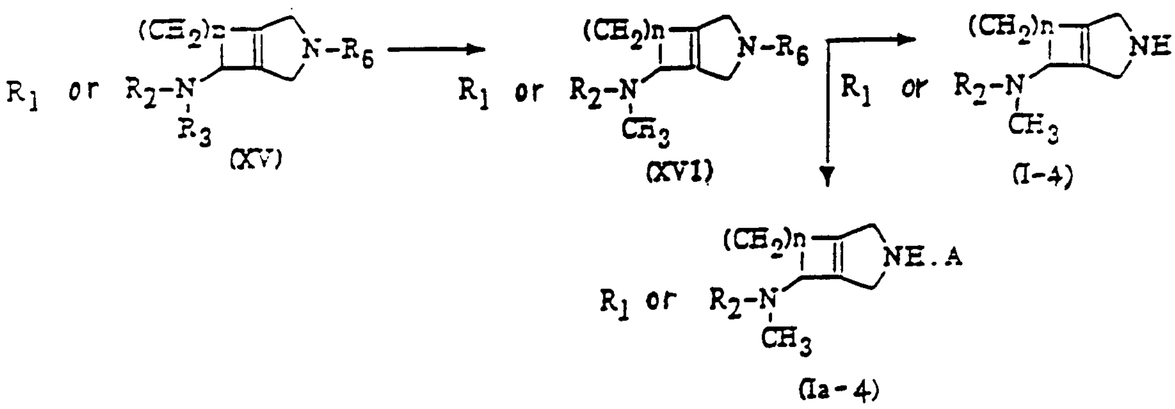

(XV) (XVI) (I-4) (Ia-4)

wherein n, A, $R_2$ and $R_3$ are as defined above and $R_6$ is p-toluenesulfonyl or methanesulfonyl.

**25.** A rpocess for preparing a compound of formula (I-5) or (Ia-5), which comprises reducing $R_3$ in the compound of formula (XVII) to a methyl group in the presence of lithium aluminum hydride to give the compound of formula (XVIII) and then removing the protecting group $R_6$

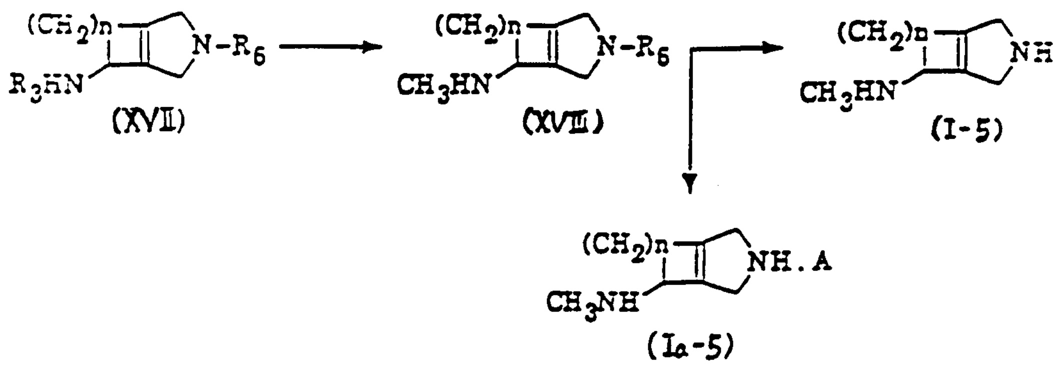

(XVII) (XVIII) (I-5) (Ia-5)

wherein n, A, $R_3$ and $R_6$ are as defined above and $R_6$ is p-toluene sulfonyl or methanesulfonyl.